# EUROPEAN PATENT SPECIFICATION

(11) **EP 1 805 323 B1**
(45) Date of publication and mention of the grant of the patent: **24.02.2010**
(21) Application number: 05812911.5
(22) Date of filing: 20.09.2005
(51) Int. Cl.: C12Q 1/26, C12Q 1/00

(54) **SYSTEMS AND METHODS FOR EVALUATING ENZYME COMPETENCY**
SYSTEME UND VERFAHREN ZUR AUSWERTUNG DER WIRKSAMKEIT VON ENZYMEN
SYSTÈMES ET PROCÉDÉS D'ÉVALUATION DE L'EFFICACITÉ ENZYMATIQUE

(30) Priority: 20.09.2004 US 611514 P
(43) Date of publication of application: 11.07.2007
(73) Proprietor: University of Florida Research Foundation, Inc., Gainesville, FL 32611 (US)
(72) Inventor: MELKER, Richard, J., Gainesville, FL 32605-3247 (US); MOREY, Timothy, E., Gainesville, FL 32608 (US); PROKAI, Laszlo, Mansfield, TX 76063 (US); DENNIS, Donn, M., Gainesville, FL 32608-4189 (US)
(74) Representative: Zwicker, Jörk
(86) International application number: PCT/US2005/033883
(87) International publication number: WO 2006/034370

(56) References cited:
- US-A- 5 100 779
- US-B1- 6 284 219
- KIVISTO K T ET AL: "Use of probe drugs as predictors of drug metabolism in humans" JOURNAL OF CLINICAL PHARMACOLOGY 1997 UNITED STATES, vol. 37, no. 1 SUPPL., 1997, pages 40S-48S, XP008059768 ISSN: 0091-2700
- TANAKA E ET AL: "In vivo function tests of hepatic drug-oxidizing capacity in patients with liver disease" JOURNAL OF CLINICAL PHARMACY AND THERAPEUTICS, vol. 22, no. 4, August 1997 (1997-08), pages 237-249, XP002367120 ISSN: 0269-4727
- KRECIC-SHEPARD MARY ELLEN ET AL: "In vivo comparison of putative probes of CYP3A4/5 activity: Erythromycin, dextromethorphan, and verapamil" CLINICAL PHARMACOLOGY AND THERAPEUTICS, vol. 66, no. 1, July 1999 (1999-07), pages 40-50, XP002367121 ISSN: 0009-9236
- DUCHARME J ET AL: "Dextromethorphan as an in vivo probe for the simultaneous determination of CYP2D6 and CYP3A activity" JOURNAL OF CHROMATOGRAPHY B : BIOMEDICAL APPLICATIONS, ELSEVIER SCIENCE PUBLISHERS, NL, vol. 678, no. 1, 29 March 1996 (1996-03-29), pages 113-128, XP004044158 ISSN: 0378-4347
- PRUIM J ET AL: "Selection criteria for liver donation: a review." TRANSPLANT INTERNATIONAL : OFFICIAL JOURNAL OF THE EUROPEAN SOCIETY FOR ORGAN TRANSPLANTATION. 1993, vol. 6, no. 4, 1993, pages 226-235, XP008059765 ISSN: 0934-0874
- DATABASE MEDLINE [Online] US NATIONAL LIBRARY OF MEDICINE (NLM), BETHESDA, MD, US; January 1997 (1997-01), ZHAO R ET AL: "[A gas chromatographic method for determination of Butyraldehyde as a product of butanol biotransformation in rat liver]" XP002367124 Database accession no. NLM15739443 & SE PU = CHINESE JOURNAL OF CHROMATOGRAPHY / ZHONGGUO HUA XUE HUI. JAN 1997, vol. 15, no. 1, January 1997 (1997-01), pages 73-74, ISSN: 1000-8713
- BATE N J ET AL: "Molecular characterization of an Arabidopsis gene encoding hydroperoxide lyase, a cytochrome P-450 that is wound inducible" PLANT PHYSIOLOGY, AMERICAN SOCIETY OF PLANT PHYSIOLOGISTS, ROCKVILLE, MD, US, vol. 117, no. 4, August 1998 (1998-08), pages 1393-1400, XP002128564 ISSN: 0032-0889
- BILLARD THIERRY: "Synthetic applications of beta -fluoroalkylated alpha , beta unsaturated carbonyl compounds" CHEM. EUR. J.; CHEMISTRY - A EUROPEAN JOURNAL JAN 23 2006, vol. 12, no. 4, 23 January 2006 (2006-01-23), pages 974-979, XP002367122
- MURPHY M.J. ET AL: 'ACUTE TOXICITY OF FLUORINATED ETHER ANESTHETICS ROLE OF 2 2 2 TRI FLUORO ETHANOL AND OTHER METABOLITES' TOXICOLOGY AND APPLIED PHARMACOLOGY vol. 71, no. 1, 01 January 1983, pages 84 - 92, XP008098022
- ROMASCHIN A. ET AL: 'THE METABOLISM OF 3 DEOXY-3-FLUORO-D GLUCOSE BY LOCUSTA-MIGRATORIA AND SCHISTOCERCA-GREGARIA' CAN J BIOCHEM vol. 55, no. 4, 1977, pages 369 - 375, XP008098023
- DONG C. ET AL: 'Crystal structure and mechanism of a bacterial fluorinating enzyme' NATURE no. 6974, 05 February 2004, pages 561 - 565, XP002288659

## Description

### Background of Invention

A leading cause of morbidity and mortality in the U.S. is adverse drug reactions (ADRs) or adverse drug events (ADEs). It is estimated that over 2.5 million ADRs occur each year in hospitals, ambulatory settings and nursing homes, leading to over 100,000 deaths. Many ADRs are the result of faulty drug metabolism, particularly those involving the cytochrome P450 (CYP) enzyme system.

In general, ADRs can be attributed to abnormally high drug levels. Abnormal enzyme function, either too much enzyme activity (such as induction of enzyme mass by drugs) or too little enzyme activity (such as genetics, drug inhibition) can cause harm by producing sub-therapeutic and supratherapeutic drug levels, respectively. ADRs caused by defective enzyme function can be grouped into three categories: 1) genetic deficiencies in enzyme function (such as polymorphisms); 2) drug-induced inhibition or augmentation of enzyme function; and 3) competition for enzyme "attention" by multiple drugs which are substrates for that particular enzyme.

Pharmacogenomics is a new discipline focused on determining the genetic basis of polymorphisms and predicting how individuals will metabolize particular drugs based on their individual "enzyme" make-up. Unfortunately, even if the genetic polymorphisms of every individual are known, many conditions, particularly organ dysfunction (such as liver disease) or xenobiotic-induced alterations in key enzymes metabolizing various therapeutic agents can lead to ADRs, even in individuals with a normal genetic makeup who under most circumstances would metabolize such drugs normally.

US 6,284,219 B1 relates to analytical methods that allow for diagnosis and management of therapy for diseases involving discrete biochemical pathways. In these methods a labelled tracer probe is administered to a subject, a labelled product of the action of enzyme is measured, and the appearance and concentration of the product are related to the disease condition of interest.

US 5,100,779 relates to a method for determining the function of a unique cytochrome P-450 activity of the type which uniquely catalyses the N-demethylation of erythromycin in a patient's body. In the method, a known quantity of N-methyl carbon labelled erythromycin solution is ingested, samples of exhaled breath are collected at known intervals of time, and the quantity of a labelled carbon metabolite originating from the labelled erythromycin is detected.

K.T. Kivistö et al. (J. Clin. Pharmacol., 1997, vol. 37, pp. 40S-48S) outline the influence of genetic and environmental factors on CYP-mediated, oxidative drug metabolism and discuss the role of different probe drugs in assessing the *in vivo* activity of CYP1A2 and CYP3A4.

E. Tanaka & D.D. Breimer (Journal of Clinical Pharmacy and Therapeutics, 1997, vol. 22, pp. 237-249) present a review on *in vivo* function tests of hepatic drug-oxidizing capacity in patient with liver disease. They discuss non-selective and selective P450 function tests.

M.E. Krecic-Shepard et al. (Clinical Pharmacology & Therapeutics, July 1999, vol. 66, no. 1, pp. 40-50) study and compare the suitability of erythromycin, dextromethorphan and verapamil as probes of CYP3A4/5 activity.

J. Ducharme et al. (J. Chromatogr. B, 1996, vol. 678, pp. 113-128) study whether dextromethorphan is a suitable probe for the simultaneous determination of CYP2D6 and CYP3A activity.

J. Pruim et al. (Transpl. Int., 1993, vol. 6, pp. 226-235) present an overview of the criteria used for the selection of liver donors. A lidocaine-MEGX test is mentioned as one test to assess the donor liver.

R. Zhao et al. (Chinese Journal of Chromatography, Jan 1997, vol. 15, pp. 73-74) present a gas chromatographic method for determination of butyraldehyde as a product of butanol biotransformation in rat liver.

N.J. Bate et al. (Plant Physiol., 1998, vol. 117, pp. 1393-1400) present a molecular characterization of an Arabidopsis gene encoding hydroperoxide lyase, a cytochrome P-450 that is wound inducible. Enzyme activity is analysed by determining the volatile reaction products trans-2-hexenal and n-hexanal by gas chromatography-mass spectrometry.

M.J. Murphy et al. (Toxicol. Appl. Pharmacol., 1983, vol. 71, pp. 84-92) study the toxicity of fluorinated ether anaesthetics in rats and focus on the role of the metabolite 2,2,2-trifluoroethanol.

A. Romaschin et al. (Can. J. Biochem. 1977, vol. 55, pp. 369-375) study the metabolism of 3-deoxy-3-fluoro-D-glucose in two locust species. Two enzymes responsible for the accumulation of the metabolite 3-deoxy-3-fluoro-D-glucitol are identified.

C. Dong et al. (Nature, 5 February 2004, vol. 427, pp. 561-565) describe the sequence and 3D structure of 5'-fluoro-5'-deoxyadenosine synthase from *Streptomyces cattleya.* The enzyme catalyses the formation of 5'-fluoro-5'-deoxyadenosine from S-adenosyl-L-methionine and fluoride.

The major enzymatic system responsible for the majority of ADRs is the CYP enzyme system. There are a large number of P450 enzymes, but the CYP3A4 enzyme accounts for metabolism of approximately 50% of all drugs, with CYP2D6 accounting for metabolism of approximately 33% of drugs. Although these are the major enzymes responsible for drug metabolism, certain other factors can also contribute to drug metabolism and homeostatic well being of humans in a manner that may be unrelated to drug metabolism (such as changes in enzyme function as a consequence of disease or changes in enzyme function causing disease).

Understanding the competency of enzyme systems, particularly the CYP system, in real time is extremely important in determining whether a patient is lively to suffer an ADR, has a disease associated with defects in specific enzyme function, or has an enzyme defect that is likely to cause pathophysiology. To date, no technology has adequately addressed this need for point-of-care assessment of enzyme system competency.

### Brief Summary

The subject invention provides systems and methods in which the function of an enzyme of interest is assessed after administering a parent molecular entity (PME) to a patient, which is known to be a substrate for said enzyme of interest. In one embodiment of the invention, PMEs have attached thereto target markers that are released upon enzymatic cleavage, wherein the target markers are fluorinated compounds. Alternatively, PMEs themselves, or metabolites of PMEs, generate target markers, wherein the target markers are fluorinated compounds. After PME administration, a sample of bodily fluid from the patient is analyzed to detect any target markers that are released and/or generated after PME interaction with a target enzyme.

The systems and methods of the subject invention can be extended to assessing enzyme activity of organs and tissues, including the gut (for assessing enzyme competency in relation to clinical treatment) and the liver. For example, one embodiment of the invention enables the assessment of functional capacity and viability of liver under consideration for transplantation into another separate patient with hepatic failure.

The liver possesses two major functions, synthesis and detoxification. In general, a number of medical laboratory tests are available to measure the synthetic function of the liver due to the large number of essential proteins (*e.g*., pre-albunun, albumin, glycoprotein, many factors necessary for thrombosis) manufactured and secreted by hepatic tissue. These proteins can be measured directly or by functional tests (*e.g*., prothrombin time) to assess synthetic function. Unfortunately, the ability of the liver to detoxify substances is more difficult to ascertain, but may be determined with global tests such as direct and total bilirubin concentrations.

Accordingly, the subject invention provides systems and methods for measuring the functional capacity of the liver to detoxify substances. In one embodiment, the ability of the liver from a potential donor to metabolize CYP substrates is assessed after administration of the PME to said potential donor. Target markers from the PME are released only upon appropriate metabolism by the liver. Detection/quantification of the target marker(s) in a sample of the donor's bodily fluid using a sensor of the invention signals a liver suitable for transplantation to a donor. The subject invention enables transplant teams to rapidly and accurately determine, at least in part, if the potential donor's liver (or any other organ) has adequate functional capacity to be useful to a transplant recipient with all the acute and chronic risks associated with solid organ transplantation.

The subj ect invention also provides systems and methods for in vitro assessment of enzymatic function. In one embodiment, a PMB (*i.e*., molecule metabolized by a relevant P450 system) is introduced ex vivo to a substrate comprising an enzyme of interest. Preferably, a known aliquot of a PME would be placed into contact with the enzyme system within a closed container. Assessment of enzyme activity can be rapidly, precisely, and accurately determined by analyzing concentration of target markers released from the substrate. For example, a sensor of the invention can be used to detect/quantify the target marker(s) present in the headspace of the closed container to assess the amount of functional enzymes present in the substrate. Based on this data, subsequent experimental data using the substrate could be normalized to the mass of functional enzyme present to allow more conclusive and reproducible results from experimental data using P450 enzyme systems.

The subj ect invention is particularly advantageous in the medical field in that enzymatic competency can be readily assessed using the systems and methods of the invention. For example, certain embodiments of the invention are designed to be employed at the point of care, such as in emergency rooms, operating rooms, hospital laboratories and other clinical laboratories, doctor's offices, in the field, or in any situation in which a rapid and accurate result is desired.

In particular, the subject invention concerns the use of a sensor that can distinguish, detect, and quantify target markers in a sample of bodily fluid (for example, blood, exhale breath, urine) to assess the competency of an enzyme of interest. Enzyme competency assessment, according to the subject invention, involves tracking the concentration of target marker(s) in bodily fluid sample(s) that reflect blood level concentrations of PME(s) or PME(s) metabolites. In certain instances, the mere identification of target markers in bodily fluid samples provides important information regarding enzymatic competency. For example, if a target marker is absent from bodily fluid samples after PME administration, it can be an indication that the PME was not properly metabolized by the patient.

In one embodiment, the concentration of target marker(s) is tracked over time to provide a 'fingerprint' of enzymatic function. This fingerprint can then be compared against fingerprints known to represent normal enzymatic function to determine the competency of the enzymatic system of interest. In another embodiment, the fingerprint can be compared to an earlier fingerprint of the patient to observe for any changes in enzyme function due to drugs, malnutrition, hepatic disease, *etc*.

By enabling enzyme competency assessment for PMEs, the invention is particularly advantageous in that adverse drug reactions (ADRs) caused by abnormal enzyme function can be detected and addressed. Since target enzymes for most pharmaceutical drugs are well known, PMEs based on pharmaceutical drugs are especially useful in assessing ADRs related to impaired drug metabolism.

More importantly, the present invention enables the determination of individualized therapy in a point of care setting. For example, a patient who would normally demonstrate enzymatic competency for certain therapeutic compounds (such as anesthetics or Warfarin) may, in certain emergent situations (for example, with liver failure or abnormal cytochrome P450 function), not be able to properly metabolize such compounds. According to the present invention, it is assessed immediately whether the patient can properly metabolize an anesthetic PME without inducing an adverse drug reaction after administering said anesthetic PME to the patient during an emergent situation. In another example, where an individual has a multiple drug regimen, a clinician using the subject technology can easily assess whether the addition (or subtraction) of a drug may induce an adverse drug reaction as well as assess the patient's ability to properly metabolize a newly added drug.

Potential types of PMEs of the subject technology include: (1) drugs approved by the Federal Drug Administration (FDA) for use in humans to treat conditions or diseases; (2) compounds generally recognized as safe (GRAYS) by the FDA that are metabolized by an enzyme system of interest; and (3) new chemical entities (NCEs) not previously approved by the FDA.

### Brief Description of Drawings

**Figure 1** is a graph illustrating the ability of SAW sensors to detect propofol in exhaled breath.
**Figure 2** is a graph illustrating the results of a study regarding propofol pharmacodynamics.
**Figure 3** is a graph illustrating the total frequency shift for three SAW sensors plotted against predicted propofol blood concentrations.
**Figure 4** is a graph illustrating the total frequency shift for three SAW sensors plotted against measured propofol blood concentrations.
**Figure 5** is a GC-MS chromatograph showing absence of propofol peak before propofol was administered to the subject.
**Figure 6** is a GC-MS chromatograph showing propofol peak with calculated effect site concentration of 0.5µg/mL (abundance = 80,000 counts).
**Figure 7** is a GC-MS chromatograph with propofol peak at calculated effect site concentration of 1.0µg/mL (abundance = 2,200,000 counts).
**Figure 8** is a GC-MS chromatograph with propofol peak at calculated effect site concentration of 0.3 µg/mL (abundance =1 1,200,000 counts).
**Figure 9** is a schematic diagram of CYP 3A4 metabolizing verapamil analogue to liberate a marker easily detected in exhaled breath.
**Figure 10A** is a schematic diagram of CYP 2D6 metabolizing a dextromethorphan analogue to liberate a marker easily detected in exhaled breath.
**Figures 10B** **and** **10C** are illustrations of preparatory schemes for synthesizing a dextromethorphan analogue in accordance with the invention.
**Figure 11** is an illustration of a preparatory scheme for synthesizing a Norverapamil analogue in accordance with the subject invention.

### Detailed Disclosure

The subject invention concerns systems and methods for use in the assessment of a wide variety of enzyme systems. The subject invention is particularly advantageous in that diagnosis of abnormal enzyme function can be made in real-time, with a point of care evaluation. Because abnormal enzyme competency is often associated with a multitude of acquired and/or genetic diseases, diagnosis of incompetent enzyme systems can be useful in preventing ADRs and also in evaluating other clinical conditions that are related to the abnormal enzymatic system.

### Definitions

As used herein, "point of care" refers to real time enzymatic competency assessment (such as diagnostic testing) that can be done in a rapid time frame so that the resulting assessment is performed faster than comparable tests that do not employ the system and methods of the invention. In addition, with the method and devices provided herein, assessment can be performed rapidly and on site in locales where rapid and accurate results are required. Point of care includes, but is not limited to: emergency rooms, operating rooms, hospital laboratories and other clinical laboratories, doctor's offices, in the field, or in any situation in which a rapid and accurate result is desired.

The term "bodily fluid," as used herein, refers to a mixture of molecules obtained from a patient. Bodily fluids include, but are not limited to, exhaled breath, whole blood, blood plasma, urine, semen, saliva, lymph fluid, meningeal fluid, amniotic fluid, glandular fluid (for example, breast milk), sputum, feces, sweat, mucous, vaginal fluid, ocular humors, and cerebrospinal fluid. Bodily fluid also includes experimentally separated fractions of all of the preceding solutions or mixtures containing homogenized solid material, such as feces, tissues, and biopsy samples.

The term "marker," as used herein, refers to a molecule or compound that is innocuous to the patient at doses relevant to the invention and detectable by means of its physical or chemical properties. As such, markers are detectable by a number of sensor technologies known in the art including, but not limited to, flow cytometers, conductive and semiconductive gas sensors; mass spectrometers; infrared (IR), ultraviolet (UV), visible, or fluorescence spectrophotometers; gas chromatography; conductive polymer gas sensor technology; surface acoustic wave gas sensor technology; immunoassay technology; microsphere (bead) array technology; molecularly imprinted polymeric film technology; microelectromechanical systems (MEMS); and amplifying fluorescent polymer (AFP) sensor technology.

The markers of the invention include federally approved products categorized as GRAYS ("generally recognized as safe") as well as other compounds not formally designated as GRAS, either an FDA approved drug or a new chemical entity, that have suitable toxicological and physicochemical properties to be detected in accordance with the systems and methods of the subject invention. For example, a marker of the invention can be a volatile marker of a known cytochrome P450 substrate (such as CYP3A4 substrate such as verapamil or a verapamil analog), which is detectable in bodily fluids, in particular blood and breath.

A "patient," as used herein, describes an organism, including mammals, to which treatment with the compositions according to the present invention is provided. Mammalian species that benefit from the disclosed systems and methods include, but are not limited to, apes, chimpanzees, orangutans, humans, monkeys; and domesticated animals (such as pets) such as dogs, cats, mice, rats, guinea pigs, and hamsters.

As used herein, the term "pharmaceutically acceptable carrier" means a carrier that is useful in preparing a pharmaceutical composition that is generally compatible with the other ingredients of the composition, not deleterious to the patient, and neither biologically nor otherwise undesirable, and includes a carrier that is acceptable for veterinary use as well as human pharmaceutical use. "A pharmaceutically acceptable carrier" as used in the specification and claims includes both one and more than one such carrier.

The invention described herein utilizes an administered parent molecular entity (PME), which is a substrate for at least one targeted enzyme system whose function is to be assessed, wherein attached to the PME is a target marker that is released upon enzymatic interaction with the PME. In one embodiment, an administered PME is metabolized by more than one target enzyme. In a related embodiment, the PME is selective for one particular enzyme over others.

As contemplated herein, at least one PME can be used in conjunction with medicinal compounds or therapies. For example, according to the subject invention, at least one PME is concurrently administratable with therapeutic methods for treating clinical conditions (for example, increasing physical activity, changing dietary consumption, decreasing/eliminating alcohol consumption and smoking); drugs (such as insulin for treating diabetes); and/or hormone replacement therapy. With such embodiments, the subject invention enables determination of enzymatic function in view of factors outside of PME activity.

Further embodiments of the invention utilize more than one administered PME to assess the competency of multiple enzyme systems. The PMEs are separately or concurrently administratable to a patient. For example, according to the subject invention, more than one PME can be provided in admixture, such as in a pharmaceutical composition. Alternatively, more than one PME can be provided as separate compounds, such as, for example, separate pharmaceutical compositions administered consecutively, simultaneously, or at different times. Preferably, if more than one PME are separately administratable, they are not administratable so distant in time from each other that multiple enzymatic system competencies cannot be accurately assessed.

In addition to assessing enzyme competency with regard to medicinal compounds and therapies, the systems and methods of the invention can be extended to assess enzyme activity in organs and tissues, such as the liver. For example, one embodiment of the invention assesses the functional capacity and viability of liver under consideration for transplantation into another separate patient with hepatic failure. Preferably, the functional capacity of the liver to detoxify substances can be assessed by (1) sampling and assessing patient bodily fluid for the presence and/or concentration of target marker(s) that were generated from a PME upon being metabolized by the liver after administering said PME to a potential donor; and (2) determining, based on the assessed target marker detection/concentration, whether the potential donor's liver is suitable for transplantation. Preferably, a sample of the donor's exhaled breath is analyzed, which would allow transplant teams to rapidly and accurately determine, at least in part, if the potential donor's liver has adequate functional capacity to be useful to a transplant recipient with all the acute and chronic risks associated with solid organ transplantation.

In a related embodiment, assessment of organ stability for transplantation can be accomplished ex vivo using the systems and methods described herein. For example, a sample from an explanted liver, artificial liver, or other hepatic tissue (such as microsomes) can be placed in contact with a PME, wherein the PME would generate a target marker upon being metabolized. Preferably, a microsome sample is placed in a closed container with a PME and the headspace of the container is subsequently analyzed using a sensor to detect/quantify the target marker(s) present in the headspace. The concentration of target marker(s) present in the headspace provides information regarding the functional capacity of the liver sample (explanted liver, artificial liver, *etc*.) and suitability for transplantation.

One aspect of the subject invention involves reconstitution and use of both recombinant and non-recombinant CYP enzymes in the laboratory. Currently, the activity of CYP enzyme systems relies on International Unit standardization that allows for batch-to-batch changes and differences between laboratories worldwide, which does not provide a uniform standard for accurately assessing CYP enzyme activity. The subject invention provides an elegant, accurate, and precise way to determine the amount of active P450 enzyme fractions in a system/sample. In one embodiment, a known aliquot of PME (such as a molecule metabolized by the relevant P450 system) is placed into contact with the target enzyme system/sample within a closed container, wherein the PME generates a target marker upon being metabolized by the target enzyme system/sample. Subsequent sampling and assessment of the container headspace for target (volatile) markers would allow rapid, precise, and accurate quantification of the activity of the enzymes present in the sample. Based on this data, subsequent experimental data using the system/sample can be normalized to the mass of functional enzyme present to allow more conclusive and reproducible results from experimental data using P450 enzyme systems.

After administering more than one PME, where the PMEs are different substrate probes for different enzymes, the subject invention could assess the competency of several enzymes (or enzyme systems) simultaneously and in a real-time/point-of-care environment. Specifically, after PME interaction with target enzyme(s), markers that are released and that characterize PME concentrations in blood are distinguished, detected, and quantified using a sensor of the invention to provide a global assessment of multiple enzymatic function (*e.g*., all major cytochrome P450 functions). According to the subject invention, a target marker can be detectable in bodily fluid samples (such as exhaled breath) using the sensor of the invention.

PMEs, according to the subject invention, are administrable via a number of delivery routes including, but not limited to, sublingual, gastrointestinal (for example, oral, rectal), vaginal, intravenous, subcutaneous, transdermal, intramuscular, topically (for example, on the eye), nasal, pulmonary, and others. Depending upon the sensitivity and/or specificity of a sensor device of the invention, the dose of PME administered may be low (<1 ng/kg body weight), high (>1 gm/kg), or a value intermediate between the two.

According to the invention, target markers that can be detected using a sensor of the invention include volatile markers that may or may not be radiolabeled and detected with a portable device in a real-time point of care manner, as well as radiolabel markers alone. In a related embodiment, the target marker is a chemical moiety (such as fluorine atom). As contemplated herein, the chemical moiety can be attached to a PME, which has a known enzymatic pathway, in a manner that will allow for ease of target marker release into bodily fluids after enzyme-interaction. In a preferred embodiment, the target marker is such that it is easily recognized over other products (such as other endogenous products or "background" noise).

To assess enzyme competency, the presence of target marker(s) in a sample of bodily fluid is measured after PME administration. Because a known enzymatic interaction with a PME causes the release of a target marker, the detection of a target marker is an easy indicator of enzymatic competency. Specifically, the presence of a target marker in a bodily fluid sample readily indicates whether the PME has been metabolized. For example, with certain PMEs, a target marker can be attached to a site that is cleaved by a known enzyme. Thus, upon PME-enzymatic cleavage, the target marker is released for detection in a bodily fluid sample.

In certain embodiments, the concentration of target marker(s) in a given bodily fluid sample is assessed over time and compared against known concentrations of PME(s) and/or metabolites of PME that represent normal enzymatic function. Generally, where the concentration of target marker(s) represents the concentration of PME(s) in blood, the quantity of target marker(s) in bodily fluid sample(s) will be reduced in a characteristic time-dependent manner that can be characterized as a "fingerprint" of enzymatic function.

To establish a fingerprint of normal enzymatic function, after administering a specific PME that is a substrate for a target enzyme whose function is to be assessed to a patient known to demonstrate normal enzymatic function with regard to the PME, a bodily fluid sample obtained from said patient is exposed to a sensor to distinguish, detect, and quantify a target marker representative of PME and/or PME metabolite concentration in blood. The data acquired by the sensor is then used to create a fingerprint of normal enzymatic function, which is the basis for comparison in assessing enzymatic competency.

In another embodiment, a fingerprint of normal enzymatic function for a specific PME that was administered to a patient is compared to a fingerprint that was previously obtained from the same patient for the same or different PME. In doing so, any changes in enzyme function due to drugs, malnutrition, hepatic disease, *etc*. can readily be observed.

In another embodiment, the sensors of the invention provide information regarding the detected target markers to a processor, which can quantify, and trend target marker presence in exhaled breath over time. After administering a PME to the patient, the present invention provides the steps of, after a suitable period of time, analyzing exhaled breath of the patient for concentration of target marker(s) (for example, unbound substances, active metabolites, or inactive metabolites); where the presence and/or concentration of target marker(s) in the bodily fluid sample indicates a characteristic of metabolism of the agent in the subject. The method further includes providing results from the analysis either to the user to appropriately control patient treatment regimen based on the results (such as in cases where PME is representative of an intravenous medicine, *etc*.).

By using different routes of PME administration, dose-dependent abnormalities in enzyme function can be assessed (*e.g*., cytochrome P450 function in gut and oral administration as well as intravenous administration). In a related embodiment, after administering a PME to a patient the patient's exhaled breath is subsequently analyzed for detection and/or concentration of target marker(s), which provides information regarding patient gut absorption of the PME. Data generated by a sensor regarding target marker(s) detection and/or concentration can be analyzed using a processor to provide information useful in prescribing an appropriate patient treatment regimen. Preferably, the detection and/or concentration of target marker(s) in exhaled breath provides information regarding PME and cytochrome P450 enzyme interaction in the gut. Alternatively, detection and/or concentration of target marker(s) in exhaled breath provides information regarding PME and cytochrome P450 enzyme interaction in other areas of the body, including the liver.

In certain embodiments, results from processor analysis of exhaled breath are provided to a software program. The software program utilizes the results to develop appropriate medication dosages, which are automatically applied to the treatment regimen (for example, where the processor not only monitors target marker presence but also monitors and controls patient medication distribution/administration).

Where desired, a neural network system can be applied to the system of the invention to: (1) establish a fingerprint of normal enzymatic function; (2) identify abnormal enzymatic function; and/or (3) identify appropriate treatment based on enzymatic function.

According to the subject invention, a neural network system can perform nonlinear least squares fit to a given data set. The data set consists of examples of correct input/output pairs, whose features the neural net will "learn" during training. The inputs may consist of continuous quantities (for example, age, height, weight), or fuzzy inputs. The latter may assure a continuous range of values, but only have well-defined meanings for a discrete set of values. For example, the property of having an incompetent CYP enzymatic system could be modeled with a fuzzy input, with the discrete values 0 and 1 indicating the presence or absence of this property, but intermediate values could be used for a person with some incompetent enzymatic function characteristics.

Given a list of examples of correct input/output pairs (such as concentrations of target markers in breath representative of normal enzymatic function for a PME), a neural net of the invention can be trained to identify normal versus abnormal enzymatic function for every PME by systematically varying any/all free parameters (weights) to minimize any chi-squared error in modeling the training data set. Once these optimal weights have been determined, the trained net can be used as a model of the training data set to test for accuracy. For example, inputs from the training data are fed to the neural net, the net output will be roughly the correct output contained in the training data. Moreover, multiple applications of simple nonlinear function and multiple linear combinations of intermediate quantities can be included in the neural network system to account for inputs provided to the neural net for which no examples appeared in the training data. Such applications enable the neural net to produce output based on features extracted from related input/output pairs in the training data.

In accordance with the present invention, the training data set can include a variety of variables for inputs/outputs including, but not limited to, age, weight, height, concentrations of target marker detectable in bodily fluids, treatment to be used based on target marker concentration, diagnosis of enzymatic function (for example, incompetent CYP enzymatic system, genetic disorder based on enzymatic function) based on target marker concentration, and rate of metabolism for PME based on target marker concentration.

Yet another embodiment of the invention is a kit for point-of-care assessment of a patient's enzymatic competency to specific medications. The kit includes a sample of a parent molecular entity, to which at least one target marker is attached; and at least one sensor. In certain embodiments, the sensor includes a communication means (for example, communication wires, wireless communication capability) that enables communication between the sensor and a processor. The kit can further include software that is applied to the processor for use in analyzing information provided by the sensor(s).

According to the subject invention, "normal" enzymatic competency or function will vary depending on the patient. In a general sense, "normal" enzymatic competency or function is defined as a population parameter whereas abnormal is defined as outside of that population parameter or not sufficient to allow homeostasis of the patient.

Three types of PME(s) are embodied within the subject invention: Type 1) FDA Approved Drugs; Type 2) GRAS Compounds; and Type 3) New Chemical Entities (NCEs). According to the subject invention, Type 1 or FDA Approved Drugs are drugs previously approved by the Food and Drug Administration for use in humans to treat conditions or diseases. GRAS Compounds are those compounds that are generally recognized as safe by the FDA for consumption. Finally, NCEs are non-FDA approved compounds, which are designed to be highly selective or relatively selective substrates for specific enzyme targets. Concentrations of any of these NCEs/PMEs or metabolic product of these NCEs/PMMs in blood can be calculated by analyzing a bodily fluid sample for target marker(s) using a sensor device of the invention.

In the preferred embodiment, PME(s) of Types 1 through 3 contain target markers that are chemical moieties (such as fluorine atoms) that make their detection particularly sensitive and specific in exhaled breath. A related embodiment of the invention uses conventional drugs that are exclusively metabolized by a particular enzyme system and making alterations in the chemical structure of those drugs to create Type 3 NCEs that, upon being metabolized, generate a target marker detectable in bodily fluids. In a related embodiment, detectable chemical moieties (target markers) are incorporated into the drug to produce NCEs that would generate the target marker(s) after the drug is metabolized by a particular enzyme system. Preferably, the target marker(s) is incorporated into the drug so that the drug metabolite is detectable in bodily fluids. By attaching target markers (such as detectable chemical moieties) to drugs to form NCEs, enzymatic competency can be assessed using the subject invention.

In another embodiment of the invention, chemical moieties are incorporated into Type 1 or 2 PMEs, both of which have known enzymatic pathways. Using the subject invention, target markers released, as a result of the metabolism of Type 1 or 2 PMEs, are easily recognized over other PME degradation products.

A preferred embodiment of the invention utilizes chemical moieties that are volatile as target markers detectable in bodily fluids, preferably exhaled breath. The subject invention enables the determination of enzyme competency for a patient prescribed with more than one drug. This is particularly important in critically ill patients and in patients, such as the elderly, who are prescribed and are taking a number of drugs simultaneously. For example, it is well established that the incidence of ADRs rises exponentially when 4 or more drugs are taken by a patient, especially elderly patients.

In cases where enzymatic competency for more than one administered medication is to be examined, a PME of each prescribed drug can be administered to a patient simultaneously. After PME administration, enzymatic competency can be readily determined by: gathering a sample of bodily fluid (such as exhaled breath); using a sensor to distinguish, detect, and quantify target markers in the sample to determine whether the PME has been metabolized. In certain embodiments, additional step of using the information provided by the sensor to establish a fingerprint; and comparing the sample fingerprint against fingerprint(s) that represent normal enzymatic function are also performed.

A hallmark of modem medicine is individualization of a total care plan for a given patient. This plan reached between physician and patient to treat a disease may be modified by multiple parameters including, but not limited to, the following: genetic abnormalities (*e.g*., polymorphisms), coexisting morbidities (for example, β-adrenergic receptor antagonist use in the setting of Type 1 diabetes mellitus), physiology (such as reduced dose or change in medication for aged or pregnant patients), concurrent treatment of multiple medications (for example, P450 inducers and/or inhibitors) with potential drug-drug interactions and ADRs, and social condition (such as financial ability to buy medication). The subject invention enables the user (such as a healthcare provider) to account for such parameters in a real-time, point-of case environment to fashion individualized total care plans for patients. This feature not only enhances patient safety from potentially catastrophic events (for example, ADRs), but also offers an unparalleled opportunity to markedly enhance medical care in general.

Sensor technology is used by the present invention to detect the presence of a marker in a bodily fluid sample. Sensors contemplated for use with the compositions and methods of the invention include, but are not limited to, surface acoustic wave (SAW) sensors (such as those disclosed in U.S. Patent Nos. 4,312,228 and 4,895,017, and Groves W.A. et al., "Analyzing organic vapors in exhaled breath using surface acoustic wave sensor array with preconcentration: Selection and characterization of the preconcentrator adsorbent," Analytica Chimica Acta, 371:131-143 (1988)); chemical sensors known in the art that use chemoselective coating applicable to the operation of the present invention (such as bulk acoustic wave (BAW) devices, plate acoustic wave devices, interdigitated microelectrode (IME) devices (such as those semi-conducting films of alkanethiol-stabilized gold nanoclusters deposited on interdigitated microelectrode arrays), optical waveguide (OW) devices, electrochemical sensors, and electrically conducting sensors); fluid sensor technology (such as commercial devices known as "artificial noses," "electronic noses," or "electronic tongues" or as disclosed in U.S. Patent Nos. 5,945,069; 5,918,257; 5,891,398; 5,830,412; 5,783,154; 5,756,879; 5,605,612; 5,252,292; 5,145,645; 5,071,770; 5,034,192; 4,938,928; and 4,992,244; and U.S. Patent Application No. 2001/0050228); semiconductive gas sensors; microelectromechanical systems (MEMS), which includes microfabricated nanomechanical cantilever sensors; mass spectrometers; IR, UV, visible, or fluorescence spectrophotometers; fiber optic microsphere sensor technology; surface resonance arrays; molecularly imprinted polymeric film sensor technology; microgravimetric sensors; thickness sheer mode sensors; apparatuses having conductive-polymer gas-sensors ("polymeric"); aptamer biosensors; and amplifying fluorescent polymer (AFP) sensors.

A variety of systems have been developed to collect and monitor exhaled breath components, particularly gases. For example, U.S. Pat. No. 6,010,459 to Silkoff describes a method and apparatus for the measurement of components of exhaled breath in humans. Various other apparatus for collecting and analyzing expired breath include the breath sampler of Glaser et al, U.S. Pat. No. 5,081,871; the apparatus of Kenny et al, U.S. Pat. No. 5,042,501; the apparatus for measuring expired breath of infants of Osborn, U.S. Pat. No. 4,202,352; the blood alcohol concentration measuring from respiratory air method of Ekstrom, U.S. Patent No. 5,971,937, and the instrument for parallel analysis of metabolites in human urine and expired air of Mitsui et al., U.S. Pat. No. 4,734,777. Pulmonary diagnostic systems including computerized data analysis components also are known, see, for example, Snow et al., U.S Pat. No. 4,796,639.

Bodily fluid sampling can be performed prior to, during, and/or after PME administration. Bodily fluid sampling and analysis using a sensor of the invention can be performed prior to, during, and after PME administration to enable trending of marker concentration. Alternatively, bodily fluid sampling and analysis can be performed during or after PME administration.

Following are examples that illustrate materials and procedures for practicing the invention. These examples should not be construed as limiting. All percentages are by weight and all solvent mixture proportions are by volume unless otherwise noted.

### Example 1-Selection of Enzymes

The following are examples of various enzymes, and corresponding compounds upon which the enzyme affects, that may be assessed in practicing the system and method of the present invention:

### CYP3A4

CYP3A4 metabolizes several drugs and dietary constituents including delavirdine, indinavir, ritonavir, saquinavir, amprena vir₇, zidovidine (AZT), nelfinavir mesylate, efavirenz, nevirapine, imiquimod, resiquimod, donezepil, lovastatin, simvastatin, pravastatin, flucastatin, atorvastatin, cerivastatin, rosuvastatin, benzafibrate, clofibrate, fenofibrate, gemfibrozil, niacin, benzodiazepines, erythromycin, dextromethorphan dihydropyridines, cyclosporine, lidocaine, midazolam, nifedipine, verapamil, and terfenadine. In addition, CYP3A4 activates environmental pro-carcinogens especially N'-nitrosonornicotine (NNN), 4-methylnitrosamino-1-(3-pyridyl-1-butanone) (NNK), 5-Methylchrysene, and 4, 4'-methylene-bis(2-chl-oroaniline)(tobacco smoke products).

### CYP2C6

CYP2C6 metabolizes several drugs including antiviral agents (such as Efavirenz, nevirapine, ritonavir, saquinovir, nelfinavir mesylate, and indinavir); psychotropic drugs (such as amiflamine, amitryptyline, clomipramine, clozapine, desipramine, haloperidol, imipramine, maprotiline, methoxyphenamine, minaprine, nortriptyline, paroxetine, perphenazine, remoxipride, thioridazine, tomoxetine, triflyperidol, zuclopenthixol, risperidone, and fluoxetine); cardiovascular agents (such as aprindine, bufuralol, debrisoquine, encainide, flecainide, guanoxam, indoramin, metoprolol, mexiletin, n-propylamaline, propafenone, propranolol, sparteine, timolol, and verapamil); and miscellaneous agents (such as chlorpropamide, codeine, dextromethorphan, methamphetamine, perhexilene, and phenformin).

### CYP2E1

CYP2E1 metabolizes several drugs and dietary constituents including isoflurane, halothane, methoxyflurane, enflurane, propofol, thiamylal, sevoflurane, ethanol, acetone, acetaminophen, nitrosamines, nitrosodimethylamine, and p-nitrophenol.

In addition, CYP2E1 activates environmental pro-carcinogens especially nitrosodimethylamine, nitrosopyrrolidone, benzene, carbon tetrachloride, and 3-hydroxypyridine (tobacco smoke product). In one study it has been shown that individuals who have high CYP2E1 (CYP2EI *5A or CYP2E1 *5B) activity are at a greater risk for gastric cancer (OR=23.6-25.7).

### CYP1A2

CYP1A2 metabolizes several drugs and dietary constituents including resiquimod, imiquimod, tacrine, acetaminophen, anti pyrine, 17 β-estradiol, caffeine, cloipramine, clozapine, flutamide (antiandogenic), imipramine, paracetamol, phenacetin, tacrine and theophylline.

In addition, CYP1A2 activates environmental pro-carcinogens, especially heterocyclic amines and aromatic amines. In one study it has been shown that individuals who are fast N-acetylators and have high CYP1a2 activity are at a greater risk for colorectal cancer (35% of cases vs. 16% of controls, OR=2.79 (P0.00-2).

### CYP2A6

CYP2A6 metabolizes several drugs including neuroleptic drugs and volatile anesthetics as well as the natural compounds, coumarin, nicotine and aflatoxin B1. In addition, CYP2A6 activates several components of tobacco smoke (for example, NNK), as well as 6-aminochrysene. The role of activation of tobacco smoke and the metabolism of nicotine have suggested a role for CYP2A6 in the development of smoking related cancers.

### CYP2C19

CYP2C19 metabolizes a variety of compounds including the tricyclic antidepressants amitriptyline, imipramine and clomipramine, the sedatives diazepam and hexobarbital, the gastric proton pump inhibitors, omeprazole, pantoprazole, and lansoprazole, as well as the antiviral nelfinavir mesylate, the antimalarial drug proguanil and the β-blocker propanolol.

### CYP2C9

CYP2C9 metabolizes a variety of compounds including S-warfarin, phenytoin, tolbutamide, tienilic acid, and a number of nonsteroidal anti-inflammatory drugs such as diclofenac, piroxicam, tenoxicam, ibuprofen, and acetylsalicyclic acid.

### Acetylcholinesterase, Butylcholinsterase, and Paroxonase

Acetylcholinesterase (often referred to simply as cholinesterase) is an enzyme that breaks down acetylcholine after it crosses the synapse between nerve cells or muscle cells. Butylcholinsterase also breaks down acetylcholine, mostly in peripheral tissue such as plasma, gut, and liver. Very little is known about paroxonase, although it has been shown to be critical in detoxifying certain forms of pesticides that are introduced into the body. All of these enzymes appear to be important in mitigating the effects of chemical warfare agents such as sarin, tabun, *etc*. Using the subject invention, patients can be screened to ascertain their level of sensitivity to such warfare agents.

### Example 2-Examples of PME Types 1, 2, and 3

### Type 1: FDA Approved Drug (Propofol)

As contemplated herein, FDA approved drugs can serve as a PME. Either the PME and/or its metabolites will be detected in a sample of bodily fluids (such as a sample of exhaled breath). This pathway is attractive because it allows use of an agent already used by a patient.

Propofol, (2,6 diisopropylphenol) is a unique anesthetic that is administered intravenously (IV), rather than by inhalation, as are traditional potent inhalation anesthetic agents. This anesthetic has a very short onset of action and an equally short offset, qualities that make it ideal for short surgical procedures. Propofol is an example of a PME that can serve as a detectable marker in bodily fluids; in particular, propofol can be directly measured in exhaled breath. The rate of disappearance in exhaled breath serves as an index of the drug's metabolism. In this case, cytochrome P450 2B6 (or CYP2B6), and to a lesser extent CYP2C9, contribute to the oxidative metabolism of propofol.

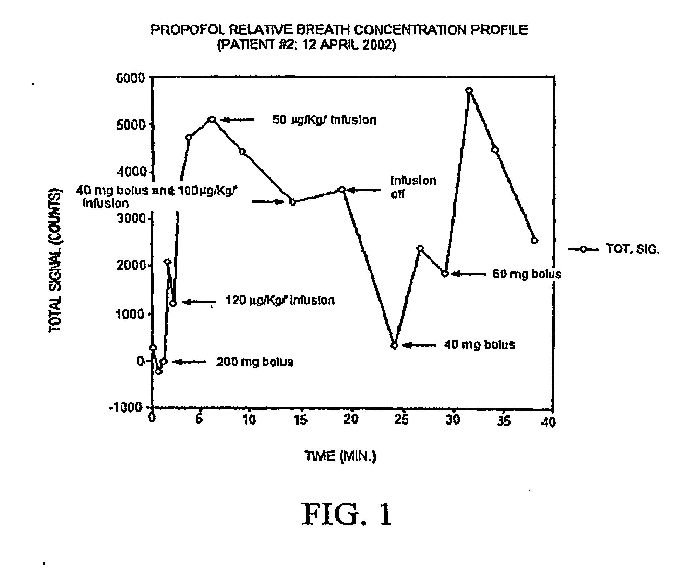

Figure 1, illustrates the ability of sensors of the invention to detect propofol in exhaled breath. Specifically, pilot studies were performed which demonstrated that propofol could be detected, even when given in low sedation concentrations, in the exhaled breath of animals and humans by using a commercial off the shelf (COTS) nerve gas detector (Hazmatcad, Microsensor Systems, Inc., Bowling Green KY). A study in the OR on a single subject demonstrated that the exhaled breath propofol concentration recorded with the Hazmatcad tracked both bolus and infusion doses of propofol.

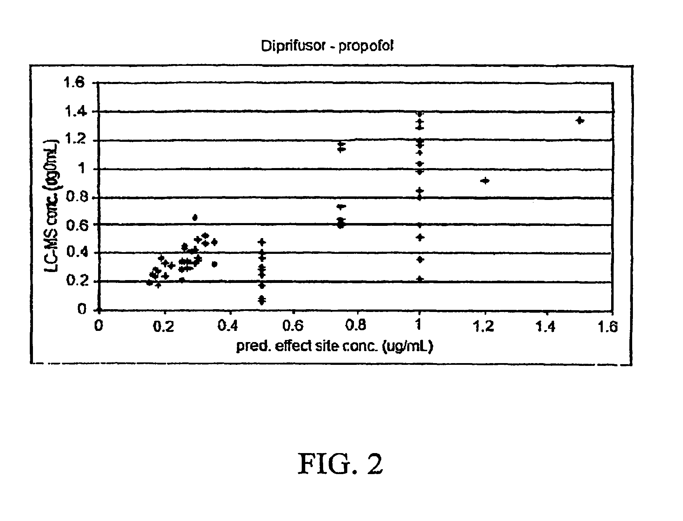

An additional pilot study was performed on a group of volunteers with normal enzymatic competency. The group of volunteers was given low sedative doses of propofol in order to evaluate its effects on thermal pain perception. The effect site concentrations of propofol was programmed using an algorithm that analyzes the pharmocodynamics of propofol (see Figure 2). Such results were compared to actual blood concentration measured with LC-MS-MS. The blood effect site concentration was changed every 15 minutes and blood samples drawn 15 minutes later. In addition, alveolar samples of exhaled breath samples were collected in Teflon bags and the propofol concentration was measured using GC-MS. Samples were collected from the Teflon bags using Solid Phase Microextraction (SPME) fibers and the remaining sample was used to measure propofol concentration using the Hazmatcad. Technical issues, including the appropriate means for collected enhaled breath samples, leaking bags, a less the optimal preconcentrator in the Hazmatcad, relatively low temperature (for propofol) used to vaporize the gas collected on the preconcentrator, and the permeability of Teflon limited value of the study determining whether exhaled breath propofol concentration correlated quantitatively with blood concentration, however, propofol was detected in all exhaled breath samples using the Hazmatcad.

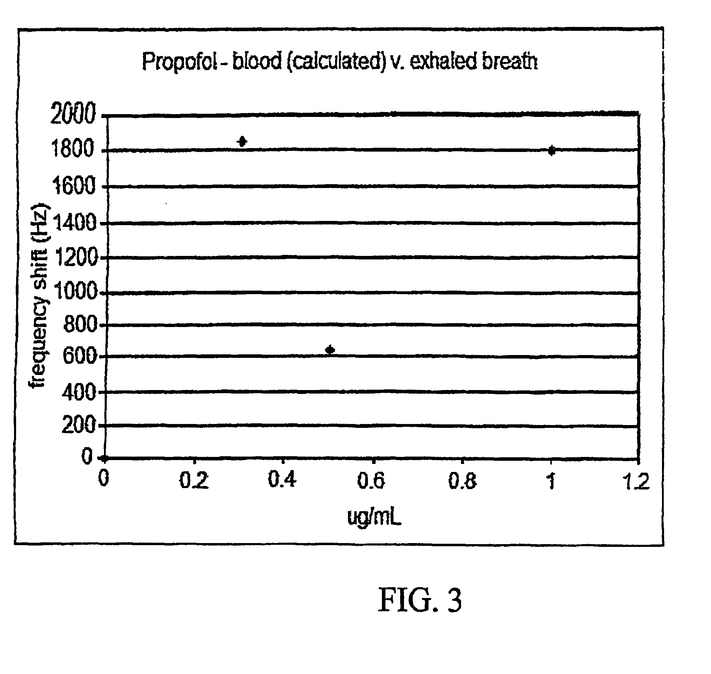

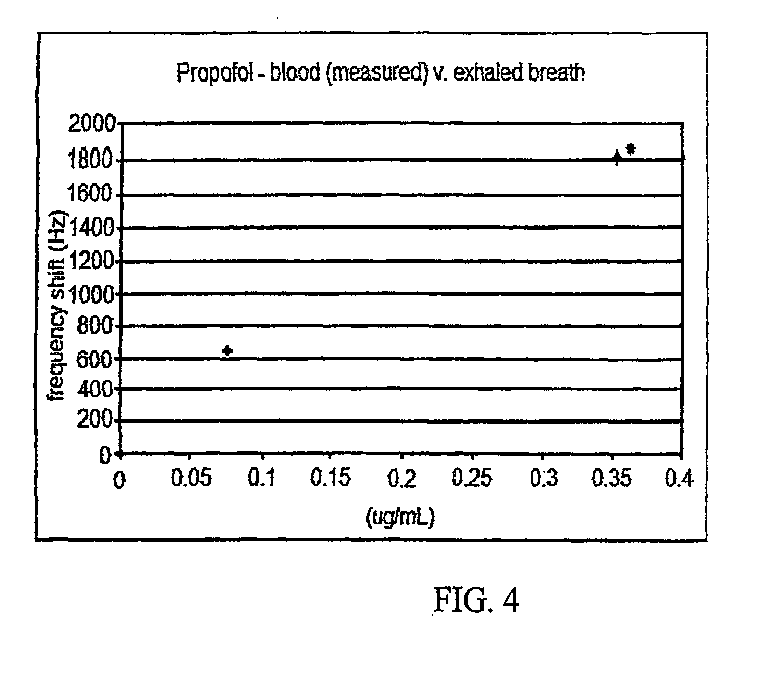

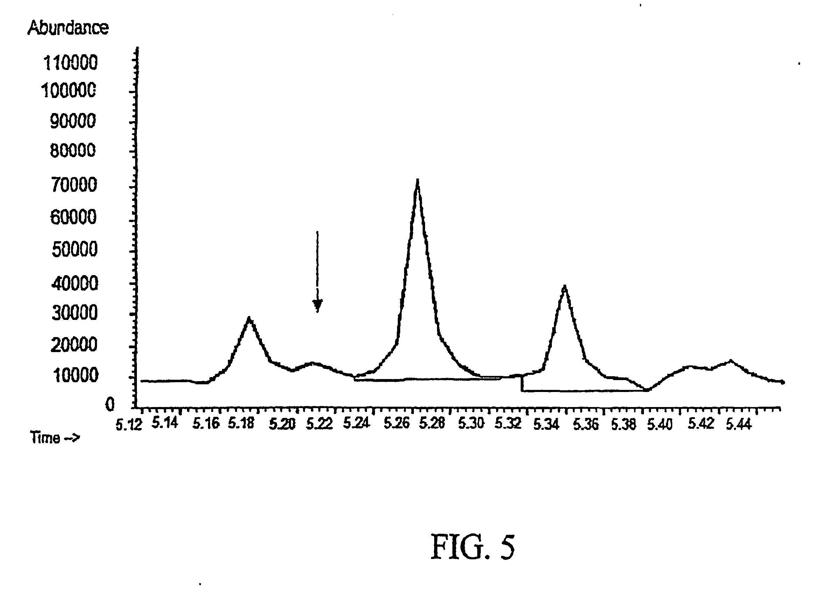

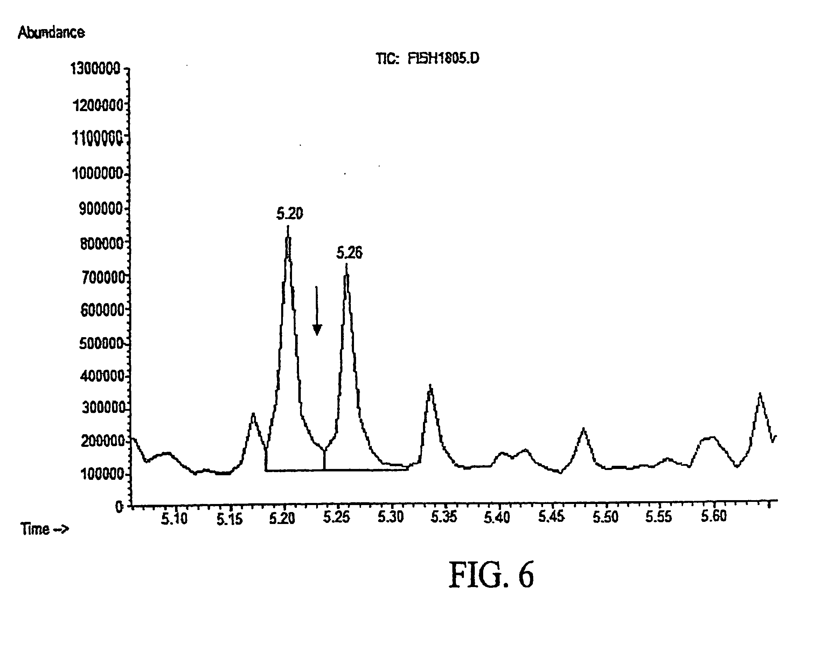

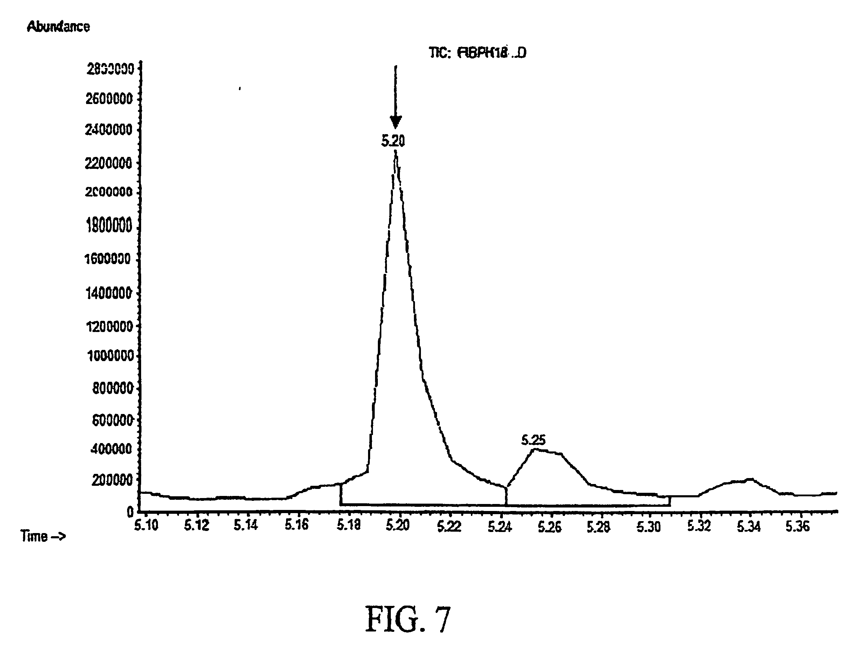

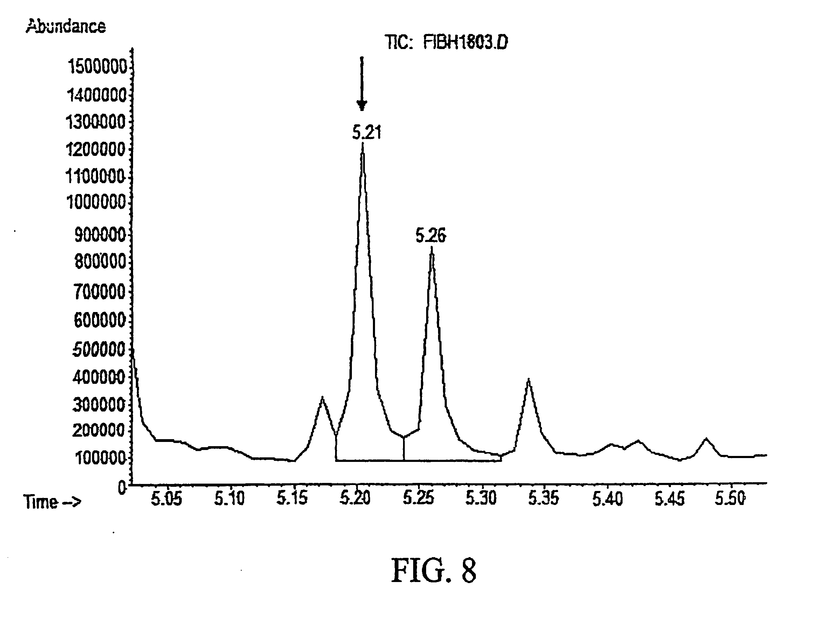

In addition, in a single subject, exhaled breath propofol was semiquantitatively measured using SPME and with the Hazmatcad. In this subject, the concentration of propofol measure by both of these techniques did correlate with the measured blood propofol concentration (see Figures 3-8).

### Type 2-GRAS Compound

In some cases a GRAS (Generally Recognized as Safe) molecule may be used as a PME and/or marker utilizing the methods and/or compositions of the invention. It is known that certain GRAS molecules have the ability to be transmitted to patient via a mucus membrane (such as gastrointestinal mucosa). Such GRAS compounds may be metabolized by an enzyme system of interest and generate a product or products that can be detected in exhaled breath. The following Table 1 provides a list of GRAS compounds that may be used in accordance with the subject invention.

| Table 1-List of Potential PMEs based on GRAS | | | | |
|---|---|---|---|---|
| DOCTYPE | DOCNUM | MAINTERM | CODE* | REGNUM |
| ASP | 311 | DibenzylEther | 000103-50-4 | 172.515 |
| ASP | 328 | Difurfuryl Ether | 004437-22-3 | |
| ASP | 445 | Ethylene Glycol Monobutyl Ether | 000111-76-2 | 175.105 175.105 176.210 177.1650 |
| ASP | 557 | Furfuryl Methyl Ether | 013679-46-4 | |
| ASP | 775 | Isoeugenyl Benzyl Ether | 000120-11-6 | 172.515 |
| ASP | 776 | Isoeugenyl Ethyl Ether | 007784-67-0 | 172.515 |
| ASP | 778 | Isoeugenyl Methyl Ether | 000093-16-3 | 172.515 |
| ASP | 1023 | Methyl Phenethyl Ether | 003558-60-9 | |
| ASP | 1097 | Beta-Naphthyl Isobutyl Ether | 002173-57-1 | |
| ASP | 3017 | Vanillyl Butyl Ether | 082654-98-6 | |
| NIL | 2028 | Dimethylethanolamine | 000108-01-0 | 173.20 175.105 175.300 |
| EAF | 3383 | Isopentylideneisopentylamine | 035448-31-8 | |

| | | | | |
|---|---|---|---|---|
| *CAS, RN, OR OTHER CODE | | | | |

The definitions of the labels that are provided in Table 1 are as follows:

| **Label** | **Definition** | |
|---|---|---|
| **DOCTYPE** | An indicator of the status of the toxicology information available for the substance in PAFA (administrative and chemical information is available on all substances) | |
| | **ASP** | Fully up-to-date toxicology information has been sought |
| | **EAF** | There is reported use of the substance, but it has not yet been assigned for toxicology literature research |
| | **NEW** | There is reported use of the substance, and an initial toxicology literature search is in progress |
| | **NIL** | Although listed as added to food, there is no current reported use of the substance and, therefore, although toxicology information may be available in PAFA, it is not being updated |
| | **NUL** | There is no reported use of the substance and there is no toxicology information available in PAFA |
| | **BAN** | The substance was formerly approved as a food additive but is now banned; there may be some toxicology data available |
| **DOCNUM** | PAFA database number of the *Food Additive Safety Profile* volume containing the printed source information concerning the substance | |
| **MAINTERM** | The name of the substance as recognized by CFSAN | |
| **CAS, RN, OR OTHER CODE** | Chemical Abstract Service (CAS) Registry Number for the substance or a numerical code assigned by CFSAN to those substances that do not have a CAS Registry Number (888nnnnnn or 977nnnnn-series) | |
| **REGNUM** | Regulation numbers in Title 21 of the U.S. Code of Federal Regulations where the chemical appears | |

### Type 3-New Chemical Entity

The following examples (for example, analogues of verapamil, dextromethorphan) could be used to test the *in vivo,* real-time, point-of-care competency of P450 subfractions 3A4 and 2D6 respectively, which are responsible for the metabolism of many drugs. Although not comprehensive, these examples also demonstrate that a current drug might be chemically modified to allow liberation of volatile products for detection or an entirely new chemical entity might be used.

### Verapamil

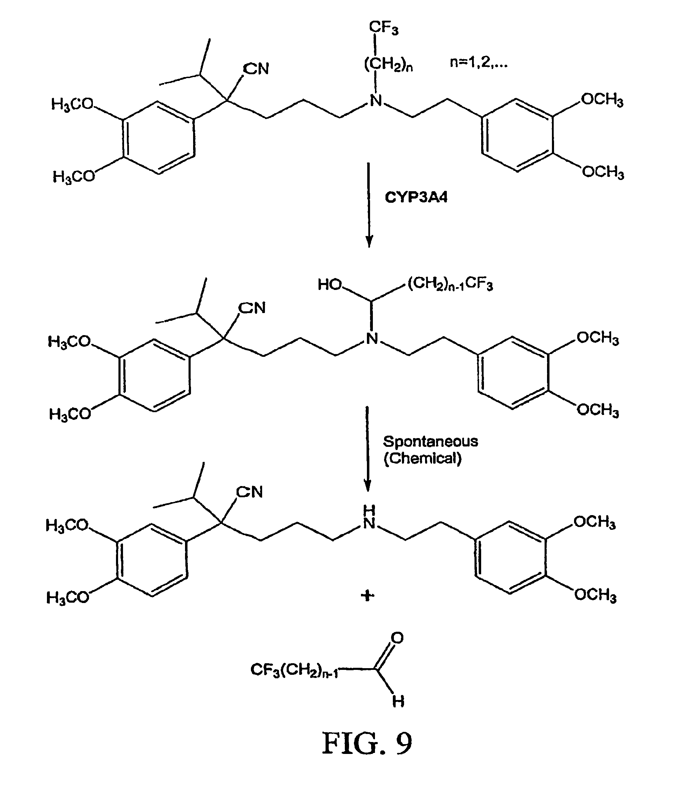

This drug is a widely prescribed calcium channel antagonist used mainly to treat essential hypertension or cardiac arrhythmias. The metabolism of verapamil is via the CYP3A4 system that metabolizes many drugs by oxidative N-dealkylation. It is commonly observed that the alkyl group lost from an amine during N-dealkylation (and from an ether during O- dealkylation) appears as an aldehyde or ketone arising from the dissociation of a carbinolamine intermediate (Brodie et al., "Enzymatic metabolism of drugs and other foreign compounds," Annu Rev. Biochem, 27:427-454 (1958); Rose and Castagnoli, "The metabolism of tertiary-amines," Med Res Rev., 3(1):73-88 (1983)). In this particular example of the present technology, verapamil is modified so that instead of the native formaldehyde being liberated due to metabolism, a non-endogenous volatile molecule is produced in a 1:1 molar ratio to the parent substrate (see Figure 9). This volatile product is transported to the lungs and excreted into the alveolar space. During exhalation, nearly all pulmonary gas is expired (except residual volume) so that a significant mass of the volatile marker can be detected in exhaled breath using various measurement methodologies (for example, SAW, infrared, fuel cell, *etc.)* In this particular situation, an inducer of cytochrome P450 3A4 increased the rate and concentration of the volatile marker in exhaled breath in an animal model above control values. In contrast, treatment with an inhibitor markedly reduced the concentration of the marker in exhaled breath. In so doing, one can rapidly test the competency of not only a general enzyme system, but also the exact CYP P450 sub-fraction responsible for specific drug metabolism, *etc.* Because of the low limit of detection for the aldehyde obtained, drug analogue is expected to be administered below the dose that would manifest pharmacological/biological activities.

Verapamil undergoes an extensive hepatic metabolism. Due to a large hepatic first-pass effect, bioavailability does not exceed 20-35% in normal subjects. Twelve metabolites have been described. The main metabolite is norverapamil and the others are various N- and 0-deakylated metabolites (Knoll Pharmaceuticals, Product Information: Isoptin SR (1984); Shomerus et al., "Physiological disposition of verapamil in man," Cardiovasc Res., 10:605-612 (1976)).

### Synthesis of a Verapamil Analogue as a CYP3A4 Substrate

In one embodiment, a process for synthesizing a verapamil analogue as a CYP3A4 substrate comprises: suspending N-Nor-(+)-verapamil hydrochloride (477 mg, 1 mmol) in 10 mL methanol, and adding sodium hydroxide (40 mg, 1 mmol) to the mixture. The precipitate is filtered off; then, the solvent is evaporated *in vacuo.* The residue is dissolved in acetonitrile (10 mL), polystyrene-bound 1,5,7-triazabicyclo[4,4,0]dec-5-ene (2 g) and 3-bromo-1,1,1-trifluoropropane (195 mg, 1.1 mmol) are added to the solution. The mixture is stirred at room temperature for 16 h. Scavenger resin (methylisocyanate bound to macroporous polystyrene resin, 2 g) is then added and the reaction mixture is agitated for a further 16 h. The solid is filtered off, washed with acetonitrile (2 x 5 mL), the filtrate is evaporated to dryness *in vacuo,* and the residue is purified by silica gel column chromatography. The purified product is then treated with diethyl ether containing 2M hydrochloric acid to obtain it in a salt form.

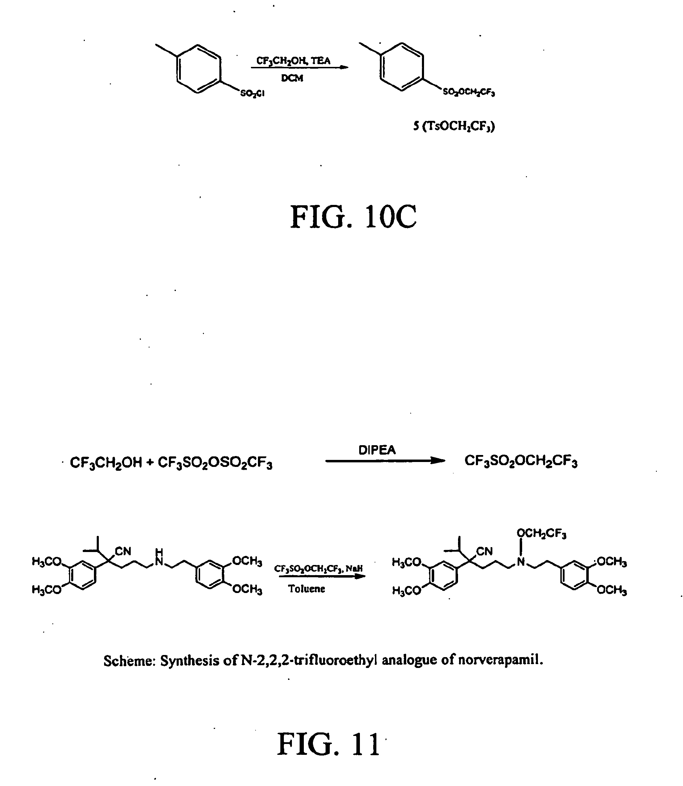

In another embodiment of the invention, an analogue of Norverapamil (N-(2,2,2-trifluoroethyl)norverapamil) is synthesized (see Figure 11). In this embodiment, Norverapamil hydrochloride (40 mg, 0.084 mmol) is dissolved in water (8 mL) and treated with K₂CO₃ to reach pH 10. The mixture is extracted with CH₂Cl₂ (3x8mL). The combined organic extracts are dried over Na₂SO₄, filtered, and the solvent is removed at reduced pressure to yield norverapamil free base. After dissolving the norverapamil in toluene (4 mL), NaH is added to the mixture and stirred for 4 hours at 70°C. Trifluoroethyl triflate (100 µL) is then added to the sodium salt of norverapamil and stirred overnight under reflux. The mixture is poured into water and extracted with diethyl ether (3×10mL). The combined organic extracts are dried over Na₂SO₄, filtered and the solvent is removed under reduced pressure to get an oily product (17 mg, 39% yield). This oil is dissolved in ethanol and treated with concentrated HCl. The hydrochloride is obtained by evaporating the solvent in vacuo.

One method for preparing the trifluoroethyl triflate (also known as 2,2,2-trifluoroethyl triflate) involves trapping 2,2,2-trifluoroethanol (1.77g, 17.7mmol, 1.27mL) in diisopropylethylamine (DIPEA, 2.30g, 17.8mmol, 3.11mL) under N₂. After dropwise addition of triflic anhydride (5g, 17.7mmol, 3mL) at 0°C, the mixture is allowed to warm to room temperature and, then, stirred for 3 hours under reflux. The product, trifluoroethyl triflate (3.2g, 78% yield) is obtained by distillation at 120°C in oil bath.

### Dextromethorphan

Dextromethorphan (3 Methoxy-17-methylmorphinan hydrobromide monohydrate; MW 370.3) is the d isomer of levophenol, a codeine analogue and opioid analgesic- The main clinical use of this agent is as an antitussive.

There is a clear first pass metabolism and it is generally assumed that the therapeutic activity is primarily due to its active metabolite, dextrophan (Silvasti et al., "Pharmacokinetics of dextromethorphan and dextrorphan: a single dose comparison of three preparations in human volunteers, Int J Clin Pharmacol Ther Toxicol, 9:493-497 (1987); Baselt & Cravey, Disposition of Toxic Drugs and Chemicals in Man, 3rd ed. Yearbook Medical Publishers, Inc., Chicago (1982)). Genetic polymorphism has profound effects on its metabolism (Hildebrand et al., "Determination of dextromethorphan metabolizer phenotype in healthy volunteers," Eur J Clin Pharmacol, 36:315-318 (1989)). Dextromethorphan undergoes polymorphic metabolism depending on variation in cytochrome P-450 enzyme phenotype(s). The specific cytochrome P-450 enzyme is P450 2D6 (CYP2D6) (Schadel et al., "The pharmacokinetics of dextromethorphan and metabolites in humans: influence of the CYP2D6 phenotype and quinidine inhibition," J Clin Psychopharmacol, 15(4):263-269 (1995)). Fast metabolizers constitute about 84% of the population.

After a 30 mg dose, plasma levels in fast metabolizers are less than 5 ng/mL four hours post ingestion (Woodworth et al., "The polymorphic metabolism of dextromethorphan," J Clin Pharmacol, 27:139-143 (1987)). Intermediate metabolizers constitute about 6.8% of the population. After an oral dose of 30 mg plasma levels are 10 to 20 ng/mL at 4 hours and less than 5 ng/mL at 24 hours postingestion (Woodworth *et al.,* 1987). Poor metabolizers constitute 5% to 10% of the Caucasian population. The ratio of metabolite to parent drug in 8 hour urine sample is less than 10 to 1 after a 15 mg dose (Hildebrand *et al.,* 1989).

After an oral dose of 30 mg, plasma levels are greater than 10 ng/mL at 4 hours and greater than 5 ng/mL at 24 hours (Woodworth *et al.,* 1987). It is metabolized in the liver by extensive metabolizers to dextrorphan. Dextrorphan is itself an active antitussive compound (Baselt & Cravey, 1982). Only small amounts are formed in poor metabolizers (Kupfer et al., "Pharmacogenetics of dextromethorphan O-demethylation in man," Xenobiotica, 16:421-433 (1986)). Less than 15% of the dose form minor metabolites including D-methoxymorphinane and D-hydroxmorphinane (Kupfer *et al.,* 1986).

Other systems amenable to this type of competency examination include, but are not limited to, alcohol dehydrogenase, alkaline phosphatase, sulfatase, cholinesterase, glucose-6-phosphate dehydogenase, prostate specific antigen and others.

### Synthesis of Dextromethorphan Analogue as a CYP2D6 Substrate (+)-

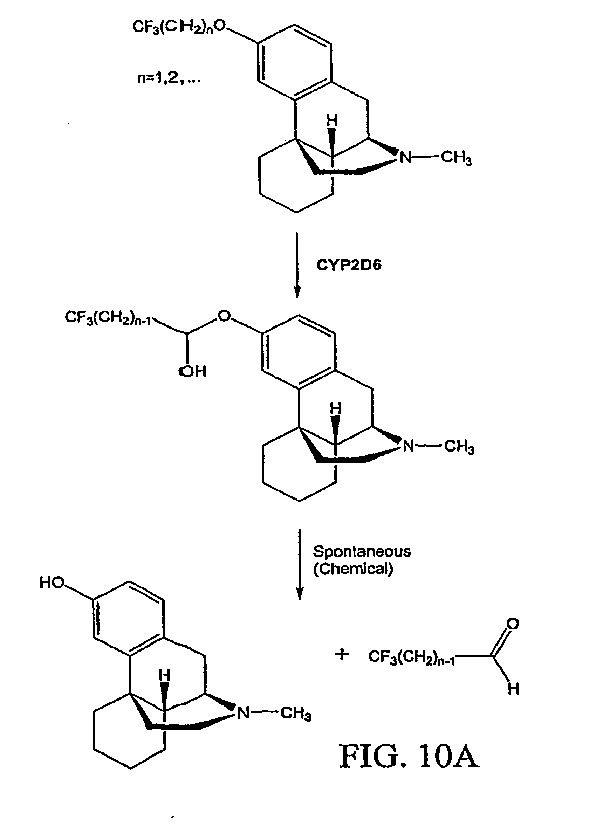

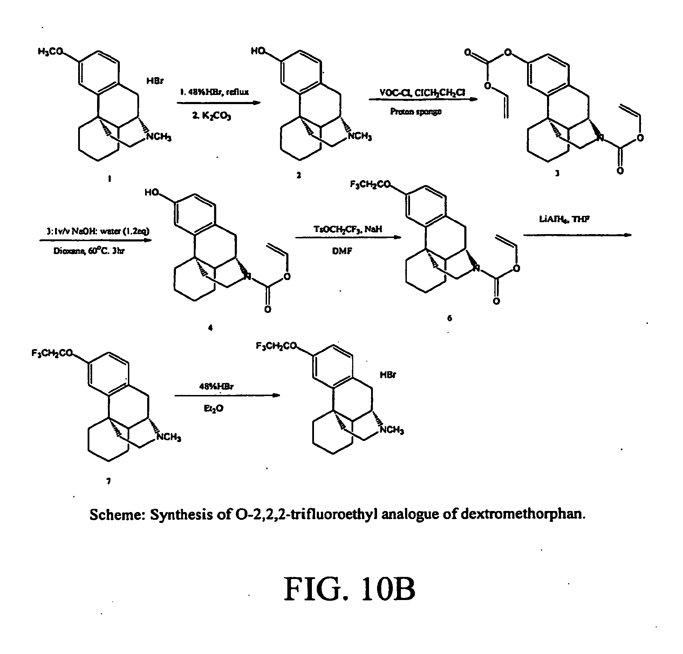

Using a similar method to verapamil modification, dextromethorphan is modified to form a product detectable in exhaled breath of humans (see end-products illustrated in Figures 10A and 10B).

In one method for synthesizing a dextromethorphan analogue (see Figure 10A), 3-Hydroxy-N-methylmorphinan tartrate salt (407 mg, 1 mmol) is dissolved in water (2 mL) and treated with saturated potassius carbonate solution until pH 10 is reached. The aqueous solution is extracted with chloroform (3x2 mL), the combined organic extract is dried over anhydrous sodium carbonate, and the solvent is evaporated at room temperature under nitrogen stream. Under dry nitrogen atmosphere, the residue is dissolved in 5 ml of dry 1,2-dichloroethane at room temperature. 1,8-Bis(dimethylamino)naphthalene (Proton Sponge, 215 mg, 1.2 mmol) and vinyl chloroformate (245 mg, 2.2 mmol) are added, and the solution is heated overnight at 60 °C. The solvent is evaporated *in vacuo,* and the residue is purified by silica gel column chromatography (dichloromethane as an eluent). After evaporation of the solvent *in vacuo,* the vinyloxycarbonyl-protected 3-hydroxymorphinan is treated with dioxane (6 mL) and water (2 mL) containing 40 mg (1 mmol) sodium hydroxide, and the solution is heated at 50 °C for 4 h. The mixture is cooled to room temperature, poured into brine and, then, extracted with 3x10 mL diethyl ether. The combined extract is dried over sodium sulfate, and the solvent is evaporated *in vacuo.* The residue is dissolved in tetrahydrofuran (5 mL), polystyrene-bound 1,5,7-triazabicyclo[4,4,0]dec-5-ene (2 g) and 3-bromo-1,1,1-trifluoropropane (195 mg, 1.1 mmol) are added to the solution. After stiffing at room temperature for 2 h, the solid is filtered off, washed with tetrahydrofuran (2 x 2 mL), and the combined filtrate is evaporated to dryness *in vacuo.* The residue is dissolved in diethyl ether (1 mL) and treated with aqueous 47% hydrogen bromide (0.1 mL). The resulting solid is filtered off and recrystallized from diethyl ether.

In another embodiment of the invention, an analogue of dextromethorphan (O-(2,2,2-trifluoroethyl)-N-methylmorphinan) is synthesized according to the scheme illustrated in Figures 10B and 10C. As illustrated in Figure 10B, dextromethorphan hydrobromide **1** (10g 28.4mmol) is dissolved in 47% aqueous hydrobromic acid (50 ml). The solution is refluxed for 18 hours. The resultant mixture is poured on crushed ice, and treated with K₂CO₃ to reach pH 10. The mixture is then extracted with chloroform (3×100mL). The combined organic extracts are washed with brine, dried over Na₂SO₄, filtered, and solvent is removed at reduced pressure to give a solid, N-methylinorphinan **2** (6g, 81 % yield).

N-methylmorphinan **2** (6g, 23.4 mmol) and Proton Sponge (1,8-bis (dimethylamino) naphthalene, 6g, 28.2 mmol) are dissolved in 1,2-dichloroethane (50 mL) at 60°C under N₂. After adding vinyl chloroformate (VOC-C1, 6g, 53 mmol), the solution is heated overnight under reflux. The resultant mixture is filtered and concentrated, and the residue is passed through a short silica gel column, eluting with CH₂Cl₂. Combination of the desired fractions followed by solvent removal gave a yellow oil, N,O-bis(vinyloxycarbonyl)morphinan 3 (5 g, 56 % yield).

N,O-bis(vinyloxycarbonyl)morphinan **3** (3.27 g, 8.5 mmol) is then dissolved in dioxane (36mL) and water (12 mL) containing 408mg (10.2 mmol) of NaOH. The solution is heated at 60°C for 3 hours. Thin layer chromatography (TLC) revealed no starting material present. The mixture is cooled to room temperature (RT), poured into brine, and extracted with ether (3×50mL). The combined ether extracts are dried over Na₂SO₄, filtered, and resultant solvent is removed under reduced pressure to give an oil, 3-Hydroxy-N-vinyloxycarbonylmorphinan **4** (2.5 g, 94 % yield).

To a rapidly stirred solution of 3-Hydroxy-N-vinyloxycarbonylmorphinan **4** (2g, 6.4mmol) in dry DMF (25 mL), NaH (60% oil dispersion, 400 mg, 10 mmol) is added under N₂. After stirring for 1 hour, 2,2,2-Trifluoroethyl-p-toluenesulfonate 5 (2.3 g, 9.1 mmol) in 10 mL of DMF is added dropwise. The mixture is stirred at RT overnight and then poured into 100 mL brine and extracted with ether (3×40mL). The combined organic extracts are dried over Na₂SO₄, filtered and the solvent was removed at reduced pressure to give an oil, 3-(2,2,2-Trifluoroethyl-N-vinyloxycarbonylmorphinan **6** (1g, 40% yield).

A scheme for synthesizing 2,2,2-Trifluoroethyl-p-toluenesulfonate 5 is illustrated in Figure 10C. p-Toluenesulfonyl chloride (4.5g, 24mmol) dissolved in CH₂Cl₂ (10ml) is added dropwise under N₂ to a solution of 2,2,2-trifluoroethanol (1.6g, 16 mmol) in 10mL CH₂Cl₂, followed by 4.5mL triethylamine (TEA) at 0°C. After completion of the TEA addition, the reaction mixture is stirred at RT for 16 hours. The mixture is concentrated, and the residue is dissolved in EtOAc (150 mL), washed with NaHCO₃ (100mL), 0.5 M Citric acid (100mL), distilled water (100mL), and dried over Na₂SO₄. Solvent is removed under reduced pressure to get the product, 2,2,2-Trifluoroethyl-p-toluenesulfonate **5** (3.2 g, 80 % yield).

To synthesize the dextromethorphan analogue O-(2,2,2-trifluoroethyl)-N-methyhnorphinan 7, 3-(2,2,2-Trifluoroethyl-N-vinyloxycarbonylmorphinan **6** (730 mg, 1.84 mmol) is dissolved in dry THF (25 mL) and stirred in ice bath for 30 min under N₂. LiAlH₄ (300 mg, 7.9 mmol) is added in small portions with rapid stirring at 0°C. After stirring overnight at room temperature, 0.3 mL water and 0.3 mL of 15% aqueous NaOH are added. The resultant mixture is poured into 75 mL of water and extracted with ether (4x30 mL). The combined organic extracts are dried over Na₂SO₄, filtered and solvent is removed at reduced pressure to give an oil (360 mg, 58% yield). The oil (60mg) is dissolved in diethyl ether (1 mL) and treated with 48% aqueous HBr (200 µL), then evaporated under high vacuum to obtain the hydrobromide salt of O-(2,2,2-trifluoroethyl)-N-methylmorphinan.

### Characteristic Properties of Verapamil and Dextromethorphan Analogues

Affinity of the trifluoroethyl analogues of verapamil and dextromethorphan to CYP3A4 and CYP2A6, respectively, is examined by measuring the inhibition of the functional activity of cDNA-expressed human CYP isoforms (2D6 or 3A4) against isoform substrates, specifically dibenzylfluorescein for 3A4 and 3-[2-(N,N-diethyl-N-methylamino)ethyl]-7-methoxy-4-methylcoumarin for 2D6 (Crespi et al., "High throughput screening for inhibition of cytochrome P450 metabolism," Med. Chem. Res., 8:457-471 (1998)). Metabolites (fluorescein for 3A4 and 3-[2-(N,N-diethyl-N-methylamino)ethyl]-7-hydroxy-4-methylcoumarin for 2D6) were determined by fluorimetric assay. IC₅₀s were determined by incubating with varying concentrations substrates (see Tables 2 and 3 below). The concentrations that yield 50% inhibition of the metabolism of the fluorescent substrate (IC₅₀s) of the parent drugs (verapamil and dextromethorphan) were used as reference values for assessing changes in affinity to the target CYP isoform.

| Table 2-The concentrations that yield 50% inhibition of the metabolism of dibenzyl-fluorescein (5-10⁻⁷ M) by human recombinant CYP3A4. | |
|---|---|
| **Compound** | **IC₅₀ (M)** |
| Ketoconazole* | 6.09·10⁻⁸ |
| Verapamil | >10⁻⁵ |
| N-(2,2,2-trifluoroethyl)norverapamil | 1.93-10⁻⁶ |

| | |
|---|---|
| *Reference inhibitor for CYP3A4. | |

| Table 3-The concentrations that yield 50% inhibition of the metabolism of 3-[2-(N,N-by diethyl-N-methylamino)ethyl]-7-methoxy-4-methylcoumarin (1.5-10⁻⁶ M) by human recombinant CYP2A6. | |
|---|---|
| **Compound** | **IC₅₀ (M)** |
| Quinidine* | 2.06-10⁻⁸ |
| Dextromethorphan | 1.82-10⁻⁶ |
| O-(2,2,2-trifluoroethyl)-N-methymorphinan | 4.25·10⁻⁷ |

| | |
|---|---|
| *Reference inhibitor for CYP2A6. | |

As illustrated in Tables 2 and 3, the 2,2,2-trifluoroethyl analogues of both verapamil and dextromethorphan display an improved affinity to the target CYP isoform when compared to the parent drugs (verapamil and dextromethorphan).

### Example 3-Selection of Sensors

The following are examples of various sensor technologies that may be utilized in practicing the method of the present invention:

### Microgravimetric Sensors

Microgravimentric sensors are based on the preparation of polymeric- or biomolecule-based sorbents that are selectively predetermined for a particular substance, or group of structural analogs. A direct measurement of mass changes induced by binding of a sorbent with a target marker can be observed by the propagation of acoustic shear waves in the substrate of the sensor. Phase and velocity of the acoustic wave are influenced by the specific adsorption of target markers onto the sensor surface. Piezoelectric materials, such as quartz (SiO₂) or zinc oxide (ZnO), resonate mechanically at a specific ultrasonic frequency when excited in an oscillating field. Electromagnetic energy is converted into acoustic energy, whereby piezoelectricity is associated with the electrical polarization of materials with anisotropic crystal structure. Generally, the oscillation method is used to monitor acoustic wave operation. Specifically, the oscillation method measures the series resonant frequency of the resonating sensor. Types of sensors derived from microgravimetric sensors include quartz crystal microbalance (QCM) devices that apply a thickness-shear mode (TSM) and devices that apply surface acoustic wave (SAW) detection principle. Additional devices derived from microgravimetric sensors include the flexural plate wave (FPW), the shear horizontal acoustic plate (SH-APM), the surface transverse wave (STW) and the thin-rod acoustic wave (TRAW).

### Conducting Polymers

Conducting polymer sensors promise fast response time, low cost, and good sensitivity and selectivity. The technology is relatively simple in concept. A conductive material, such as carbon, is homogeneously blended in a specific nonconducting polymer and deposited as a thin film on an aluminum oxide substrate. The films lie across two electrical leads, creating a chemoresistor. As the polymer is subjected to various chemical vapors, it expands, increasing the distance between carbon particles, and thereby increasing the resistance. The polymer matrix swells because analyte vapor absorbs into the film to an extent determined by the partition coefficient of the analyte. The partition coefficient defines the equilibrium distribution of an analyte between the vapor phase and the condensed phase at a specified temperature. Each individual detector element requires a minimum absorbed amount of analyte to cause a response noticeable above the baseline noise. Selectivity to different vapors is accomplished by changing the chemical composition of the polymer. This allows each sensor to be tailored to specific chemical vapors. Therefore, for most applications an array of orthogonal responding sensors is required to improve selectivity. Regardless of the number of sensors in the array, the information from them must be processed with pattern recognition software to correctly identify the chemical vapors of interest. Sensitivity concentration are reportedly good (tens of ppm). The technology is very portable (small and low power consumption), relatively fast in response time (less than 1 minute), low cost, and should be rugged and reliable

### Electrochemical Sensors

Electrochemical sensors measure a change in output voltage of a sensing element caused by chemical interaction of a target marker on the sensing element. Certain electrochemical sensors are based on a transducer principle. For example, certain electrochemical sensors use ion-selective electrodes that include ion-selective membranes, which generate a charge separation between the sample and the sensor surface. Other electrochemical sensors use an electrode by itself as the surface as the complexation agent, where a change in the electrode potential relates to the concentration of the target marker. Further examples of electrochemical sensors are based on semiconductor technology for monitoring charges at the surface of an electrode that has been built up on a metal gate between the so-called source and drain electrodes. The surface potential varies with the target marker concentration.

Additional electrochemical sensor devices include amperometric, conductometric, and capacitive immunosensors. Amperometric immunosensors are designed to measure a current flow generated by an electrochemical reaction at a constant voltage. Generally, electrochemically active labels directly, or as products of an enzymatic reaction, are needed for an electrochemical reaction of a target marker at a sensing electrode. Any number of commonly available electrodes can be used in amperometric immunosensors, including oxygen and H₂O₂ electrodes.

Capacitive immunosensors are sensor-based transducers that measure the alteration of the electrical conductivity in a solution at a constant voltage, where alterations in conductivity are caused by biochemical enzymatic reactions, which specifically generate or consume ions. Capacitance changes are measured using an electrochemical system, in which a bioactive element is immobilized onto a pair of metal electrodes, such as gold or platinum electrodes.

Conductometric immunosensors are also sensor-based transducers that measure alteration of surface conductivity. As with capacitive immunosensors, bioactive elements are immobilized on the surface of electrodes. When the bioactive element interacts with a target marker, it causes a decrease in the conductivity between the electrodes.

Electrochemical sensors are excellent for detecting low parts-per-million concentrations. They are also rugged, draw little power, linear and do not require significant support electronics or vapor handling (pumps, valves, etc.) They are moderate in cost ($50 to $200 in low volumes) and small in size.

### Gas Chromatography / Mass Spectrometry (GC/MS)

Gas Chromatography/Mass Spectrometry (GC/MS) is actually a combination of two technologies. One technology separates the chemical components (GC) while the other one detects them (MS). Technically, gas chromatography is the physical separation of two or more compounds based on their differential distribution between two phases, the mobile phase and stationary phase. The mobile phase is a carrier gas that moves a vaporized sample through a column coated with a stationary phase where separation takes place. When a separated sample component elutes from the column, a detector converts the column eluent to an electrical signal that is measured and recorded. The signal is recorded as a peak in the chromatogram plot. Chromatograph peaks can be identified from their corresponding retention times. The retention time is measured from the time of sample injection to the time of the peak maximum, and is unaffected by the presence of other sample components. Retention times can range from seconds to hours, depending on the column selected and the component. The height of the peak relates to the concentration of a component in the sample mixture.

After separation, the chemical components need to be detected. Mass spectrometry is one such detection method, which bombards the separated sample component molecules with an electron beam as they elute from the column. This causes the molecules to lose an electron and form ions with a positive charge. Some of the bonds holding the molecule together are broken in the process, and the resulting fragments may rearrange or break up further to form more stable fragments. A given compound will ionize, fragment, and rearrange reproducibly under a given set of conditions. This makes identification of the molecules possible. A mass spectrum is a plot showing the mass/charge ratio versus abundance data for ions from the sample molecule and its fragments. This ratio is normally equal to the mass for that fragment. The largest peak in the spectrum is the base peak. The GC/MS is accurate, selective and sensitive.

### Infrared Spectroscopy (FTIR, NDIR)

Infrared (IR) spectroscopy is one of the most common spectroscopic techniques used by organic and inorganic chemists. Simply, it is the absorption measurement of different IR frequencies by a sample positioned in the path of an IR beam. IR radiation spans a wide section of the electromagnetic spectrum having wavelengths from 0.78 to 1000 micrometers (microns). Generally, IR absorption is represented by its wave number, which is the inverse of its wavelength times 10,000. For a given sample to be detected using IR spectroscopy, the sample molecule must be active in the IR region, meaning that the molecule must vibrate when exposed to IR radiation. Several reference books are available which contain this data, including the Handbook of Chemistry and Physics from the CRC Press.

There are two general classes of IR spectrometers - dispersive and non-dispersive. In a typical dispersive IR spectrometer, radiation from a broadband source passes through the sample and is dispersed by a monochromator into component frequencies. The beams then fall on a detector, typically a thermal or photon detector, which generates an electrical signal for analysis. Fourier Transform IR spectrometers (FTIR) have replaced the dispersive IR spectrometer due to their superior speed and sensitivity. FTIR eliminates the physical separation of optical component frequencies by using a moving mirror Michelson interferometer and taking the Fourier transform of the signal.

Conversely, in the non-dispersive IR (NDIR) spectrometer, instead of sourcing a broad IR spectrum for analyzing a range of sample gases, the NDIR sources a specific wavelength which corresponds to the absorption wavelength of the target sample. This is accomplished by utilizing a relatively broad IR source and using spectral filters to restrict the emission to the wavelength of interest. For example, NDIR is frequently used to measure carbon monoxide (CO), which absorbs IR energy at a wavelength of 4.67 microns. By carefully tuning the IR source and detector during design, a high volume production CO sensor is manufactured. This is particularly impressive, as carbon dioxide is a common interferent and has an IR absorption wavelength of 4.26 microns, which is very close to that of CO.

NDIR sensors promise low cost (less than $200), no recurring costs, good sensitivity and selectivity, no calibration and high reliability. They are small, draw little power and respond quickly (less than 1 minute). Warm up time is nominal (less than 5 minutes). Unfortunately, they only detect one target gas. To detect more gases additional spectral filters and detectors are required, as well as additional optics to direct the broadband IR source.

### Ion Mobility Spectrometry (IMS)

Ion Mobility Spectrometry (IMS) separates ionized molecular samples on the basis of their transition times when subjected to an electric field in a tube. As the sample is drawn into the instrument, it is ionized by a weak radioactive source. The ionized molecules drift through the cell under the influence of an electric field. An electronic shutter grid allows periodic introduction of the ions into the drift tube where they separate based on charge, mass, and shape. Smaller ions move faster than larger ions through the drift tube and arrive at the detector sooner. The amplified current from the detector is measured as a function of time and a spectrum is generated. A microprocessor evaluates the spectrum for the target compound, and determines the concentration based on the peak height.

IMS is an extremely fast method and allows near real time analysis. It is also very sensitive, and should be able to measure all the analytes of interest. IMS is moderate in cost (several thousand dollars) and larger in size and power consumption.

### Metal Oxide Semiconductor (MOS) Sensors

Metal Oxide Semiconductor (MOS) sensors utilize a semiconducting metal-oxide crystal, typically tin-oxide, as the sensing material. The metal-oxide crystal is heated to approximately 400 °C, at which point the surface adsorbs oxygen. Donor electrons in the crystal transfer to the adsorbed oxygen, leaving a positive charge in the space charge region. Thus, a surface potential is formed, which increases the sensor's resistance. Exposing the sensor to deoxidizing, or reducing, gases removes the surface potential, which lowers the resistance. The end result is a sensor which changes its electrical resistance with exposure to deoxidizing gases. The change in resistance is approximately logarithmic.

MOS sensors have the advantage of being extremely low cost (less than $8 in low volume) with a fast analysis time (milliseconds to seconds). They have long operating lifetimes (greater than five years) with no reported shelf life issues.

### Thickness-Shear Mode Sensors (TSM)

TSM sensors consist of an AT-cut piezoelectric crystal disc, most commonly of quartz because of its chemical stability in biological fluids and resistance to extreme temperatures, and two electrodes (preferably metal) attached to opposite sides of the disc. The electrodes apply the oscillating electric field. Generally, TSM sensor devices are run in a range of 5-20 MHz. Advantages are, besides the chemical inertness, the low cost of the devices and the reliable quality of the mass-produced quartz discs.

### Photo-Ionization Detectors (PID)

Photo-Ionization Detectors rely on the fact that all elements and chemicals can be ionized. The energy required to displace an electron and 'ionize' a gas is called its Ionization Potential (IP), measured in electron volts (eV). A PID uses an ultraviolet (UV) light source to ionize the gas. The energy of the UV light source must be at least as great as the IP of the sample gas: For example, benzene has an IP of 9.24 eV, while carbon monoxide has an IP of 14.01 eV. For the PID to detect the benzene, the UV lamp must have at least 9.24 eV of energy. If the lamp has an energy of 15 eV, both the benzene and the carbon monoxide would be ionized. Once ionized, the detector measures the charge and converts the signal information into a displayed concentration. Unfortunately, the display does not differentiate between the two gases, and simply reads the total concentration of both summed together.

Three UV lamp energies are commonly available: 9.8, 10.6 and 11.7 eV. Some selectivity can be achieved by selecting the lowest energy lamp while still having enough energy to ionize the gases of interest. The largest group of compounds measured by a PID are the organics (compounds containing carbon), and they can typically be measured to parts per million (ppm) concentrations. PIDs do not measure any gases with an IP greater than 11.7 eV, such as nitrogen, oxygen, carbon dioxide and water vapor. The CRC Press Handbook of Chemistry and Physics includes a table listing the IPs for various gases.

PIDs are sensitive (low ppm), low cost, fast responding, portable detectors. They also consume little power.

### Surface Acoustic Wave Sensors (SAW)

Surface Acoustic Wave (SAW) sensors are constructed with interdigitated metal electrodes fabricated on piezoelectric substrates both to generate and to detect surface acoustic waves. Surface acoustic waves are waves that have their maximum amplitude at the surface and whose energy is nearly all contained within 15 to 20 wavelengths of the surface. Because the amplitude is a maximum at the surface such devices are very surface sensitive. Normally, SAW devices are used as electronic bandpass filters in cell phones. They are hermetically packaged to insure that their performance will not change due to a substance contacting the surface of the SAW.

SAW chemical sensors take advantage of this surface sensitivity to function as sensors. To increase specificity for specific compounds, SAW devices are frequently coated with a thin polymer film that will affect the frequency and insertion loss of the device in a predictable and reproducible manner. Each sensor in a sensor array is coated with a different polymer and the number and type of polymer coating are selected based on the chemical to be detected. If the device with the polymer coating is then subjected to chemical vapors that absorb into the polymer material, then the frequency and insertion loss of the device will further change. It is this final change that allows the device to function as a chemical sensor.

If several SAW devices are each coated with a different polymer material, the response to a given chemical vapor will vary from device to device. The polymer films are normally chosen so that each will have a different chemical affinity for a variety of organic chemical classes, that is, hydrocarbon, alcohol, ketone, oxygenated, chlorinated, and nitrogenated. If the polymer films are properly chosen, each chemical vapor of interest will have a unique overall effect on the set of devices. SAW chemical sensors are useful in the range of organic compounds from hexane on the light, volatility extreme to semi-volatile compounds on the heavy, low volatility extreme.

Motors, pumps and valves are used to bring the sample into and through the array. The sensitivity of the system can be enhanced for low vapor concentrations by having the option of using a chemical preconcentrator before the array. In operation, the preconcentrator absorbs the test vapors for a period of time and is then heated to release the vapors over a much shorter time span thereby increasing the effective concentration of the vapor at the array. The system uses some type of drive and detection electronics for the array. An on board microprocessor is used to control the sequences of the system and provide the computational power to interpret and analyze data from the array.

SAW sensors are reasonably priced (less than $200) and have good sensitivity (tens of ppm) with very good selectivity. They are portable, robust and consume nominal power. They warm up in less than two minutes and require less than one minute for most analysis. They are typically not used in high accuracy quantitative applications, and thus require no calibration. SAW sensors do not drift over time, have a long operating life (greater than five years) and have no known shelf life issues. They are sensitive to moisture, but this is addressed with the use of a thermally desorbed concentrator and processing algorithms.

### Amplifying Fluorescent Polymer Technology

Sensors can use fluorescent polymers that react with volatile chemicals as sensitive target marker detectors. Conventional fluorescence detection normally measures an increase or decrease in fluorescence intensity or an emission wavelength shift that occurs when a single molecule of the target marker interacts with an isolated chromophore, where the chromophore that interacts with the target marker is quenched; the remaining chromophores continue to fluoresce.

A variation of this approach is the "molecular wire" configuration, as described by Yang and Swager, J. Am. Chem. Soc., 120:5321-5322 (1998) and Cumming et al., IEEE Trans Geoscience and Remote Sensing, 39:1119-1128 (2001). In the molecular wire configuration, the absorption of a single photon of light by any chromophore will result in a chain reaction, quenching the fluorescence of many chromophores and amplifying the sensory response by several orders of magnitude. Sensors based on the molecular wire configuration have been assembled for detecting explosives (see Swager and Wosnick, MRS Bull, 27:446-450 (2002).

### Fiber Optic Microsphere Technology

Fiber optic microsphere technology is based upon an array of a plurality of microsphere sensors (beads), wherein each microsphere belongs to a discrete class that is associated with a target marker, that is placed on an optical substrate containing a plurality of micrometer-scale wells (see, for example, Michael et al., Anal Chem, 71:2192-2198 (1998); Dickinson et al., Anal Chem., 71:2192-2198 (1999); Albert and Walt, Anal Chem, 72:1947-1955 (2000); and Stitzel et al., Anal Chem, 73:5266-5271 (1001)). Each type of bead is encoded with a unique signature to identify the bead as well as its location. Upon exposure to a target marker, the beads respond to the target marker and their intensity and wavelength shifts are used to generate fluorescence response patterns, which are, in turn, compared to known patterns to identify the target marker.

### Interdigitated Microelectrode Arrays (IME)

Interdigitated microelectrode arrays are based on the used of a transducer film that incorporates an ensemble of nanometer-sized metal particles, each coated by an organic monomolecular layer shell (see, for example, Wohltjen and Snow, Anal Chem, 70:2856-2859 (1998); and Jarvis et al., Proceedings of the 3rd Intl Aviation Security Tech Symposium, Atlantic City, NJ, 639-647 (2001)). Such sensor devices are also known as metal-insulator-metal ensembles (MIME) because of the combination of a large group of colloidal-sized, conducting metal cores separated by thin insulating layers.

### Microelectromechanical Systems (MEMS)

Sensor technology based on MEMS integrate mechanical elements, sensors, actuators, and electronics on a common silicon substrate for use in detecting target markers (see, for example, Pinnaduwage et al., Proceedings of 3rd Intl Aviation Security Tech Symposium, Atlantic City, NJ, 602-615 (2001); and Lareau et al., Proceedings of 3rd Intl Aviation Security Tech Symposium, Atlantic City, NJ, 332-339 (2001)).

One example of sensor technology based on MEMS is microcantilever sensors. Microcantilever sensors are hairlike, silicon-based devices that are at least 1,000 times more sensitive and smaller than currently used sensors. The working principle for most microcantilever sensors is based on a measurement of displacement. Specifically, in biosensor applications, the displacement of a cantilever-probe is related to the binding of molecules on the (activated) surface of the cantilever beam, and is used to compute the strength of these bonds, as well as the presence of specific reagents in the solution under consideration (Fritz, J. et al., "Translating biomolecular recognition into nanomechanics," Science, 288:316-318 (2000); Raiteri, R. et al., "Sensing of biological substances based on the bending of microfabricated cantilevers," Sensors and Actuators B, 61:213-217 (1999)). It is clear that the sensitivity of these devices strongly depends on the smallest detectable motion, which poses a constraint on the practically vs. theoretically achievable performance.

One examples of microcantilever technology uses silicon cantilever beams (preferably a few hundred micrometers long and 1µm thick) that are coated with a different sensor/detector layer (such as antibodies or aptamers). When exposed to a target marker, the cantilever surface absorbs the target marker, which leads to interfacial stress between the sensor and the absorbing layer that bends the cantilever. Each cantilever bends in a characteristic way typical for each target marker. From the magnitude of the cantilever's bending response as a function of time, a fingerprint pattern for each target marker can be obtained.

Microcantilever sensors are highly advantageous in that they can detect and measure relative humidity, temperature, pressure, flow, viscosity, sound, ultraviolet and infrared radiation, chemicals, and biomolecules such as DNA, proteins, and enzymes. Microcantilever sensors are rugged, reusable, and extremely sensitive, yet they cost little and consume little power. Another advantage in using the sensors is that they work in air, vacuum, or under liquid environments.

### Molecularly Imprinted Polymeric Film

Molecular imprinting is a process of template-induced formation of specific molecular recognition sites (binding or catalytic) in a polymeric material where the template directs the positioning and orientation of the polymeric material's structural components by a self-assembling mechanism (see, for example, Olivier et al., Anal Bioanal Chem, 382:947-956 (2005); and Ersoz et al., Biosensors & Bioelectronics, 20:2197-2202 (2005)). The polymeric material can include organic polymers as well as inorganic silica gels. Molecularly imprinted polymers (MIPs) can be used in a variety of sensor platforms including, but not limited to, fluorescence spectroscopy; UV/Vis spectroscopy; infrared spectroscopy; surface plasmon resonance; checmiluminescent adsorbent assay; and reflectometric interference spectroscopy. Such approaches allow for the realization of highly efficient and sensitive target marker recognition.

## Claims

1. A method for real-time, point of care determination of enzymatic system competency, the method comprising the steps of:
a) exposing *in vitro* a sensor to a bodily fluid from a patient for whom competency for at least one enzymatic system is to be determined, wherein said sensor can distinguish, detect, and quantify at least one target marker in said bodily fluid, wherein said at least one target marker is a fluorinated compound, wherein said bodily fluid has been obtained from said patient after administering at least one parent molecular entity to said patient, and wherein said at least one target marker was generated from said at least one parent molecular entity; and
b) based on the presence of the target marker, establishing whether the patient exhibits competency for the at least one enzymatic system.

2. The method of claim 1, wherein the target marker is volatile.

3. The method of claim 1, wherein the parent molecular entity is a substrate for the at least one enzymatic system, wherein the parent molecular entity is selected from the group consisting of: an FDA approved drug; a GRAS compound; and a new chemical entity.

4. The method of claim 1, wherein the bodily fluid is selected from the group consisting of exhaled breath, whole blood, blood plasma, urine, semen, saliva, lymph fluid, meningal fluid, amniotic fluid, glandular fluid, sputum, feces, sweat, mucous, vaginal fluid, ocular humors, and cerebrospinal fluid.

5. The method of claim 4, wherein the bodily fluid is exhaled breath.

6. The method of claim 1, wherein the sensor is selected from the group consisting of: metal-insulator-metal ensemble (MIME) sensors, cross-reactive optical microsensor arrays, fluorescent polymer films, surface enhanced raman spectroscopy (SERS), diode lasers, selected ion flow tubes, metal oxide sensors (MOS), bulk acoustic wave (BAW) sensors, colorimetric tubes, infrared spectroscopy, gas chromatography, semiconductive gas sensor technology; mass spectrometers, fluorescent spectrophotometers, conductive polymer gas sensor technology; aptamer sensor technology; amplifying fluorescent polymer (AFP) sensor technology; microcantilever technology; molecularly polymeric film technology; surface resonance arrays; microgravimetric sensors; thickness sheer mode sensors; or surface acoustic wave gas sensor technology.

7. The method of claim 1, wherein the enzymatic system is the cytochrome P450 system.

8. The method of claim 7, wherein enzymatic system is the cytochrome P450 3A4 system, wherein the parent molecular entity is an analogue of verapamil, and wherein the target marker is a metabolite of verapamil.

9. The method of claim 8, wherein the analogue of verapamil is N-(2,2,2-trifluoroethyl)norverapamil.

10. The method of claim 7, wherein enzymatic system is the cytochrome P450 2D6 system, wherein the parent molecular entity is an analogue of dextromethorphan, and wherein the target marker is a metabolite of dextromethorphan.

11. The method of claim 10, wherein the analogue of dextromethorphan is O-(2,2,2-trifluoroethyl)-N-methylmorphinan.

12. The method of claim 1, further comprising the step of determining whether the patient will suffer an adverse drug reaction based on the competency of the at least one enzymatic system.

13. The method of claim 1, further comprising the step of establishing a first fingerprint for competent activity of the at least one enzymatic system, assessing the concentration of the target marker in the bodily fluid sample over time to establish a second fingerprint, and determining whether the patient has a disease based on the comparison of the first fingerprint to the second fingerprint.

14. The method of claim 1, further comprising the step of determining whether the patient will exhibit pathophysiology based on the competency of the at least one enzymatic system.

15. The method of claim 1, wherein the parent molecular entity is administrable using a route selected from the group consisting of: sublingual; gastrointestinal; vaginal; intravenous; subcutaneous; transdermal; intramuscular; topically; nasal; and pulmonary.

16. The method of claim 1, further comprising the step of constructing an individualized total care plan for the patient based on the competency of the at least one enzymatic system.

17. The method of claim 1, wherein said bodily fluid has been obtained from said patient after administering more than one parent molecular entity to said patient; and further comprising the step of prescribing beneficial dosages for more than one drug based on the competency of the enzymatic system.

18. A system for real-time, point of care determination of enzymatic system competency, said system comprising:
a) at least one parent molecular entity, wherein at least one target marker is generatable from the at least one parent molecular entity, and wherein the target marker is a fluorinated compound;
b) a sensor for detecting said target marker; and
c) a processor configured to store and analyze information provided by the sensor;
wherein
(1) the enzymatic system is the cytochrome P450 3A4 system, the parent molecular entity is an analogue of verapamil, and the target marker is a metabolite of verapamil;
or
(2) the enzymatic system is the cytochrome P450 2D6 system, the parent molecular entity is an analogue of dextromethorphan, and the target marker is a metabolite of dextromethorphan.

19. The system of claim 18, wherein the processor is also configured to store a fingerprint representative of at least one normal enzymatic function; to determine a sample fingerprint based on the data provided by the sensor; and to compare the representative fingerprint against the sample fingerprint.

20. The system of claim 18, wherein the parent molecular entity is a substrate for the at least on enzymatic system, wherein the parent molecular entity is selected from the group consisting of: an FDA approved drug; a GRAS compound; and a new chemical entity.

21. The system of claim 18, wherein the sensor is selected from the group consisting of metal-insulator-metal ensemble (MIME) sensors, cross-reactive optical microsensor arrays, fluorescent polymer films, surface enhanced raman spectroscopy (SERS), diode lasers, selected ion flow tubes, metal oxide sensors (MOS), bulk acoustic wave (BAW) sensors, colorimetric tubes, infrared spectroscopy, gas chromatography, semiconductive gas sensor technology; mass spectrometers, fluorescent spectrophotometers, conductive polymer gas sensor technology; aptamer sensor technology; amplifying fluorescent polymer (AFP) sensor technology; microcantilever technology; molecularly imprinted polymeric film technology; microgravimetric sensors; surface resonance arrays; thickness sheer mode sensors; or surface acoustic wave gas sensor technology.

22. The system of claim 18, wherein the analogue of verapamil is N-(2,2,2-trifluoroethyl)norverapamil.

23. The system of claim 18, wherein the analogue of dextromethorphan is O-(2,2,2-trifluoroethyl)-N-methylmorphinan.

24. A kit for point-of care assessment of a patient's enzymatic system competency to at least one parent molecular entity, said kit comprising:
a) a sample of said parent molecular entity, from which at least one target marker is generatable, wherein the target marker is a fluorinated compound; and
b) at least one sensor for detecting said target marker;
wherein
(1) the enzymatic system is the cytochrome P450 3A4 system, the parent molecular entity is an analogue of verapamil, and the target marker is a metabolite of verapamil;
or
(2) the enzymatic system is the cytochrome P450 2D6 system, the parent molecular entity is an analogue of dextromethorphan, and the target marker is a metabolite of dextromethorphan.

25. The kit of claim 24, wherein the sensor comprises a communication means that enables communication between the sensor and a processor.

26. The kit of claim 25, further comprising software that is applied to the processor for use in analyzing information provided by the sensor.

27. The kit of claim 24, wherein the sensor is selected from the group consisting of: metal-insulator-metal ensemble (MIME) sensors, cross-reactive optical microsensor arrays, fluorescent polymer films, surface enhanced raman spectroscopy (SERS), diode lasers, selected ion flow tubes, metal oxide sensors (MOS), bulk acoustic wave (BAW) sensors, colorimetric tubes, infrared spectroscopy, gas chromatography, semiconductive gas sensor technology; mass spectrometers, fluorescent spectrophotometers, conductive polymer gas sensor technology; aptamer sensor technology; amplifying fluorescent polymer (AFP) sensor technology; microcantilever technology; molecularly imprinted polymeric film technology; surface resonance arrays; microgravimetric sensors; thickness sheer mode sensors; or surface acoustic wave gas sensor technology.

28. The kit of claim 24, wherein the parent molecular entity is a substrate for an enzymatic system of interest, wherein the parent molecular entity is selected from the group consisting of: an FDA approved drug; a GRAS compound; and a new chemical entity.

29. The kit of claim 24, wherein the analogue of verapamil is N-(2,2,2-trifluoroethyl)norverapamil.

30. The kit of claim 24, wherein the analogue of dextromethorphan is O-(2,2,2-trifluoroethyl)-N-methylmorphinan.

31. A method for assessing potential patient organ suitability for transplantation comprising:
a) exposing *in vitro* a sensor to a bodily fluid from a patient for whom competency for at least one enzymatic system in an organ is to be determined, wherein said sensor can distinguish, detect, and quantify at least one target marker in said bodily fluid, wherein said at least one target marker is a fluorinated compound, wherein said bodily fluid has been obtained from said patient after administering at least one parent molecular entity to said patient, and wherein said at least one target marker was generated from said at least one parent molecular entity; and
b) based on the presence of the target marker, establishing whether the patient exhibits competency for the at least one enzymatic system and whether the organ is suitable for transplantation.

32. A method for *in vitro* assessment of enzyme function comprising:
a) administering in a closed container at least one parent molecular entity to a sample comprising at least one enzyme, wherein activity for the at least one enzyme is to be determined, wherein at least one target marker is generated from said at least one parent molecular entity, wherein the target marker is a fluorinated compound, and wherein the closed container comprises a headspace;
b) exposing a sensor to the headspace, wherein said sensor can distinguish, detect, and quantify the target marker in the headspace; and
c) based on the presence of the target marker, establishing whether the at least one enzyme is active.

## Patentansprüche

1. Verfahren zur patientennahen Echtzeit-Bestimmung der Kompetenz für ein enzymatisches System, wobei das Verfahren die Schritte umfasst:
a) In-Kontakt-Bringen eines Sensors *in vitro* mit einer Körperflüssigkeit von einem Patienten, für den die Kompetenz für wenigstens ein enzymatisches System bestimmt werden soll, wobei der Sensor wenigstens einen Zielmarker in der Körperflüssigkeit unterscheiden, nachweisen und quantifizieren kann, wobei der wenigstens eine Zielmarker eine fluorierte Verbindung darstellt, wobei die Körperflüssigkeit von dem Patienten erhalten wurde, nachdem wenigstens eine molekulare Ausgangsverbindung an den Patienten verabreicht worden ist, und wobei der wenigstens eine Zielmarker aus der wenigstens einen molekularen Ausgangsverbindung erzeugt wurde; und
b) Feststellen auf der Grundlage des Vorhandenseins des Zielmarkers, ob der Patient eine Kompetenz für das wenigstens eine enzymatische System aufweist.

2. Verfahren nach Anspruch 1, wobei der Zielmarker flüchtig ist.

3. Verfahren nach Anspruch 1, wobei die molekulare Ausgangsverbindung ein Substrat für das wenigstens eine enzymatische System darstellt, wobei die molekulare Ausgangsverbindung ausgewählt ist aus der Gruppe, bestehend aus: einem von der FDA zugelassenen Medikament; eine Verbindung mit GRAS-Status; und einer neuen chemischen Substanz.

4. Verfahren nach Anspruch 1, wobei die Körperflüssigkeit ausgewählt ist aus der Gruppe bestehend aus ausgeatmetem Atem, Vollblut, Blutplasma, Urin, Samenflüssigkeit, Speichel, Lymphflüssigkeit, Hirnflüssigkeit, Fruchtwasser, Drüsenflüssigkeit, Sputum, Faeces, Schweiß, Schleim, Vaginalflüssigkeit, Glaskörper und Kammerwasser des Auges und Liquor cerebrospinalis.

5. Verfahren nach Anspruch 4, wobei die Körperflüssigkeit ausgeatmeter Atem ist.

6. Verfahren nach Anspruch 1, wobei der Sensor ausgewählt ist aus der Gruppe bestehend aus: Sensoren aus Metall-Isolator-Metall-Ensembles (MIME), kreuzreaktiven optischen Mikrosensorarrays, fluoreszierenden Polymerfilmen, oberflächenverstärkter Raman-Spektroskopie (SERS), Diodenlasern, Flussröhren mit ausgewähltem Ion (selected ion flow tubes), Metalloxidsensoren (MOS), Volumenschallwellensensoren (BAW-Sensoren), colorimetrischen Röhren, Infrarotspektroskopie, Gaschromatographie, Halbleitergassensortechnik; Massenspektrometern, Fluoreszenzspektrophotometern, Gassensortechnik mit leitfähigem Polymer; Aptamer-Sensortechnik; Sensortechnik mit verstärkendem fluoreszierenden Polymer (AFP); Mikro-Ausleger-Technik; Technik mit molekularem Polymerfilm; Oberflächenresonanzarrays; mikrogravimetrischen Sensoren; Dickenschermodussensoren; oder Oberflächenschallwellengassensortechnik.

7. Verfahren nach Anspruch 1, wobei das enzymatische System das Cytochrom-P450-System ist.

8. Verfahren nach Anspruch 7, wobei das enzymatische System das Cytochrom-P450-3A4-System ist, wobei die molekulare Ausgangsverbindung ein Analogon von Verapamil ist und wobei der Zielmarker ein Metabolit von Verapamil ist.

9. Verfahren nach Anspruch 8, wobei das Analogon von Verapamil N-(2,2,2-Trifluorethyl)norverapamil ist.

10. Verfahren nach Anspruch 7, wobei das enzymatische System das Cytochrom-P450-2D6-System ist, wobei die molekulare Ausgangsverbindung ein Analogon von Dextromethorphan ist und wobei der Zielmarker ein Metabolit von Dextromethorphan ist.

11. Verfahren nach Anspruch 10, wobei das Analogon von Dextromethorphan O-(2,2,2-Trifluorethyl)-N-methylmorphinan ist.

12. Verfahren nach Anspruch 1, zusätzlich umfassend den Schritt des Bestimmens, ob der Patient eine unerwünschte Arzneimittelwirkung erleiden wird, basierend auf der Kompetenz für das wenigstens eine enzymatische System.

13. Verfahren nach Anspruch 1, zusätzlich umfassend den Schritt des Bestimmens eines ersten Fingerabdrucks für eine kompetente Aktivität des wenigstens einen enzymatischen Systems, des Bestimmens der Konzentration des Zielmarkers in der Körperflüssigkeitsprobe über die Zeit, um einen zweiten Fingerabdruck zu ermitteln, und des Bestimmens, ob der Patient eine Krankheit hat, auf der Grundlage des Vergleichs des ersten Fingerabdrucks mit dem zweiten Fingerabdruck.

14. Verfahren nach Anspruch 1, zusätzlich umfassend den Schritt des Bestimmens, ob der Patient eine Pathophysiologie aufweisen wird, auf der Grundlage der Kompetenz für das wenigstens eine enzymatische System.

15. Verfahren nach Anspruch 1, wobei die molekulare Ausgangsverbindung auf einem Weg verabreicht werden kann, der ausgewählt ist aus der Gruppe bestehend aus: sublingual; gastrointestinal; vaginal; intravenös; subkutan; transdermal; intramuskulär; topisch; nasal; und pulmonal.

16. Verfahren nach Anspruch 1, zusätzlich umfassend den Schritt des Erstellens eines individualisierten umfassenden Behandlungsplans für den Patienten auf der Grundlage der Kompetenz für das wenigstens eine enzymatische System.

17. Verfahren nach Anspruch 1, wobei die Körperflüssigkeit von dem Patienten erhalten wurde, nachdem mehr als eine molekulare Ausgangsverbindung an den Patienten verabreicht worden ist; und zusätzlich umfassend den Schritt des Verschreibens heilsamer Dosierungen von mehr als einem Medikament auf der Grundlage der Kompetenz für das enzymatische System.

18. Ein System zur patientennahen Echtzeit-Bestimmung der Kompetenz für ein enzymatisches System, wobei das System umfasst:
a) wenigstens eine molekulare Ausgangsverbindung, wobei wenigstens ein Zielmarker aus der wenigstens einen molekularen Ausgangsverbindung erzeugt werden kann und wobei der Zielmarker eine fluorierte Verbindung ist;
b) ein Sensor zum Nachweisen des Zielmarkers; und
c) einen Prozessor, der zum Speichern und Analysieren von Informationen konfiguriert ist, die durch den Sensor bereitgestellt werden;
wobei
(1) das enzymatische System das Cytochrom-P450-3A4-System ist, die molekulare Ausgangsverbindung ein Analogon von Verapamil und der Zielmarker ein Metabolit von Verapamil ist;
oder
(2) das enzymatische System das Cytochrom-P450-2D6-System ist, die molekulare Ausgangsverbindung ein Analogon von Dextromethorphan und der Zielmarker ein Metabolit von Dextromethorphan ist.

19. System nach Anspruch 18, wobei der Prozessor zusätzlich dazu konfiguriert ist, einen Fingerabdruck zu speichern, der für wenigstens eine normale enzymatische Funktion charakteristisch ist; einen Probenfingerabdruck auf der Grundlage der durch den Sensor bereitgestellten Daten zu ermitteln; und den charakteristischen Fingerabdruck mit dem Probenfingerabdruck zu vergleichen.

20. System nach Anspruch 18, wobei die molekulare Ausgangsverbindung ein Substrat für das wenigstens eine enzymatische System darstellt, wobei die molekulare Ausgangsverbindung ausgewählt ist aus der Gruppe bestehend aus: einem von der FDA zugelassenen Medikament; einer Verbindung mit GRAS-Status; und einer neuen chemischen Substanz.

21. System nach Anspruch 18, wobei der Sensor ausgewählt ist aus der Gruppe bestehend aus: Sensoren aus Metall-Isolator-Metall-Ensembles (MIME), kreuzreaktiven optischen Mikrosensorarrays, fluoreszierenden Polymerfilmen, oberflächenverstärkter Raman-Spektroskopie (SERS), Diodenlasern, Flussröhren mit ausgewähltem Ion (selected ion flow tubes), Metalloxidsensoren (MOS), Volumenschallwellensensoren (BAW-Sensoren), colorimetrischen Röhren, Infrarotspektroskopie, Gaschromatographie, Halbleitergassensortechnik; Massenspektrometern, Fluoreszenzspektrophotometern, Gassensortechnik mit leitfähigem Polymer; Aptamer-Sensortechnik; Sensortechnik mit verstärkendem fluoreszierenden Polymer (AFP); Mikro-Ausleger-Technik; Technik mit molekular geprägtem Polymerfilm; Oberflächenresonanzarrays; mikrogravimetrischen Sensoren; Dickenschermodussensoren; oder Oberflächenschallwellengassensortechnik.

22. System nach Anspruch 18, wobei das Analogon von Verapamil N-(2,2,2-Trifluorethyl)norverapamil ist.

23. System nach Anspruch 18, wobei das Analogon von Dextromethorphan O-(2,2,2-Trifluorethyl)-N-methylmorphinan ist.

24. Kit zur patientennahen Ermittlung der Kompetenz für ein enzymatisches System eines Patienten gegenüber wenigstens einer molekularen Ausgangsverbindung, wobei das Kit umfasst:
a) eine Probe der molekularen Ausgangsverbindung, aus der wenigstens ein Zielmarker erzeugt werden kann, wobei der Zielmarker eine fluorierte Verbindung ist; und
b) wenigstens ein Sensor zum Nachweisen des Zielmarkers;
wobei
(1) das enzymatische System das Cytochrom-P450-3A4-System ist, die molekulare Ausgangsverbindung ein Analogon von Verapamil und der Zielmarker ein Metabolit von Verapamil ist;
oder
(2) das enzymatische System das Cytochrom-P450-2D6-System ist, die molekulare Ausgangsverbindung ein Analogon von Dextromethorphan und der Zielmarker ein Metabolit von Dextromethorphan ist.

25. Kit nach Anspruch 24, wobei der Sensor Mittel zur Datenübertragung umfasst, die eine Datenübertragung zwischen dem Sensor und einem Prozessor gestatten.

26. Kit nach Anspruch 25 zusätzlich umfassend eine Software, die auf dem Prozessor angewendet wird, zur Verwendung bei dem Analysieren von Information, die durch den Sensor bereitgestellt wird.

27. Kit nach Anspruch 24, wobei der Sensor ausgewählt ist aus der Gruppe bestehend aus: Sensoren aus Metall-Isolator-Metall-Ensembles (MIME), kreuzreaktiven optischen Mikrosensorarrays, fluoreszierenden Polymerfilmen, oberflächenverstärkter Raman-Spektroskopie (SERS), Diodenlasern, Flussröhren mit ausgewähltem Ion (selected ion flow tubes), Metalloxidsensoren (MOS), Volumenschallwellensensoren (BAW-Sensoren), colorimetrischen Röhren, Infrarotspektroskopie, Gaschromatographie, Halbleitergassensortechnik; Massenspektrometern, Fluoreszenzspektrophotometern, Gassensortechnik mit leitfähigem Polymer; Aptamer-Sensortechnik; Sensortechnik mit verstärkendem fluoreszierenden Polymer (AFP); Mikro-Ausleger-Technik; Technik mit molekular geprägtem Polymerfilm; Oberflächenresonanzarrays; mikrogravimetrischen Sensoren; Dickenschermodussensoren; oder Oberflächenschallwellengassensortechnik.

28. Kit nach Anspruch 24, wobei die molekulare Ausgangsverbindung ein Substrat für ein enzymatisches System von Interesse darstellt, wobei die molekulare Ausgangsverbindung ausgewählt ist aus der Gruppe bestehend aus: einem von der FDA zugelassenen Medikament; einer Verbindung mit GRAS-Status; und einer neuen chemischen Substanz.

29. Kit nach Anspruch 24, wobei das Analogon von Verapamil N-(2,2,2-Trifluorethyl)norverapamil ist.

30. Kit nach Anspruch 24, wobei das Analogon von Dextromethorphan O-(2,2,2-Trifluorethyl)-N-methylmorphinan ist.

31. Verfahren zum Ermitteln der möglichen Eignung eines Patientenorgans für eine Transplantation, umfassend:
a) In-Kontakt-Bringen eines Sensors *in vitro* mit einer Körperflüssigkeit von einem Patienten, für den die Kompetenz für wenigstens ein enzymatisches System in einem Organ bestimmt werden soll, wobei der Sensor wenigstens einen Zielmarker in der Körperflüssigkeit unterscheiden, nachweisen und quantifizieren kann, wobei der wenigstens eine Zielmarker eine fluorierte Verbindung darstellt, wobei die Körperflüssigkeit von dem Patienten erhalten wurde, nachdem dem Patienten wenigstens eine molekulare Ausgangsverbindung verabreicht worden ist, und wobei wenigstens ein Zielmarker aus der wenigstens einen molekularen Ausgangsverbindung erzeugt worden ist; und
b) Feststellen auf der Grundlage des Vorhandenseins des Zielmarkers, ob der Patient Kompetenz für das wenigstens eine enzymatische System aufweist und ob das Organ zur Transplantation geeignet ist.

32. Verfahren zur *in vitro*-Bestimmung einer Enzymfunktion, umfassend:
a) Zugabe wenigstens einer molekularen Ausgangsverbindung zu einer Probe, die wenigstens ein Enzym enthält, in einem geschlossenen Behältnis, wobei die Aktivität für das wenigstens eine Enzym bestimmt werden soll, wobei wenigstens ein Zielmarker aus der wenigstens einen molekularen Ausgangsverbindung erzeugt wird, wobei der Zielmarker eine fluorierte Verbindung darstellt, und wobei das geschlossene Behältnis einen Freiraum umfasst;
b) In-Kontakt-Bringen eines Sensors mit dem Freiraum, wobei der Sensor den Zielmarker im Freiraum unterscheiden, nachweisen und quantifizieren kann; und
c) Feststellen auf der Grundlage des Vorhandenseins des Zielmarkers, ob wenigstens ein Enzym aktiv ist.

## Revendications

1. Procédé pour la détermination en temps réel au point de traitement de la compétence d'un système enzymatique, le procédé comprenant les étapes de:
a) exposition *in vitro* d'un détecteur à un fluide corporel provenant d'un patient pour lequel il convient de déterminer la compétence pour au moins un système enzymatique, tandis que ledit détecteur peut distinguer, détecter et quantifier au moins un marqueur cible dans ledit fluide corporel, tandis que ledit au moins un marqueur cible est un composé fluoré, tandis que ledit fluide corporel a été obtenu à partir du dit patient après administration d'au moins une entité moléculaire parent au dit patient, et tandis que ledit au moins un marqueur cible a été généré à partir de ladite au moins une entité moléculaire parent; et
b) sur la base de la présence du marqueur cible, établissement de ce que le patient présente ou non une compétence pour le au moins un système enzymatique.

2. Procédé selon la revendication 1, dans lequel le marqueur cible est volatil.

3. Procédé selon la revendication 1, dans lequel l'entité moléculaire parent est un substrat pour le au moins un système enzymatique, tandis que l'entité moléculaire parent est choisie dans le groupe consistant en un médicament approuvé par la FDA; un composé de type GRAS; et une nouvelle entité chimique.

4. Procédé selon la revendication 1, dans lequel le fluide corporel est choisi dans le groupe consistant en respiration exhalée, sang total, plasma sanguin, urine, sperme, salive, liquide lymphatique, liquide méningé, liquide amniotique, liquide glandulaire, crachat, matières fécales, sueur, mucus, fluide vaginal, humeurs oculaires, et liquide céphalo-rachidien.

5. Procédé selon la revendication 4, dans lequel le fluide corporel est la respiration exhalée.

6. Procédé selon la revendication 1, dans lequel le détecteur est choisi dans le groupe consistant en les détecteurs à ensemble métal-isolent-métal (MIME), les arrangements de microdétecteurs optiques à réactivité croisée, les pellicules de polymère fluorescent, la spectroscopie Raman renforcée en surface (SERS), les lasers à diode, les tubes à écoulement ionique sélectionné, les détecteurs à base d'oxyde métallique (MOS), les détecteurs à onde acoustique de volume (BAW), les tubes colorimétriques, la spectroscopie infrarouge, la chromatographie gazeuse, la technologie par détecteur à gaz semi-conducteur; les spectromètres de masse, les spectrophotomètres fluorescents, la technologie par détecteur de gaz à polymère conducteur; la technologie par détecteur aptamère; la technologie par détecteur polymère fluorescent amplificateur (AFP); la technologie par microcantilever; la technologie par pellicule polymère de type moléculaire; les arrangements pour résonance de surface; les détecteurs microgravimétriques; les détecteurs à mode de cisaillement d'épaisseur; ou la technologie par détecteur de gaz à longueur d'onde acoustique de surface.

7. Procédé selon la revendication 1, dans lequel le système enzymatique est le système du cytochrome P450.

8. Procédé selon la revendication 7, dans lequel le système enzymatique est le système du cytochrome P450 3A4, tandis que l'entité moléculaire parent est un analogue du vérapamil, et tandis que le marqueur cible est un métabolite du vérapamil.

9. Procédé selon la revendication 8, dans lequel l'analogue du vérapamil est le N-(2,2,2-trifluoroéthyl)norvérapamil.

10. Procédé selon la revendication 7, dans lequel le système enzymatique est le système du cytochrome P450 2D6, tandis que l'entité moléculaire parent est un analogue du dextrométhorphan, et tandis que le marqueur cible est un métabolite du dextrométhorphan.

11. Procédé selon la revendication 10, dans lequel l'analogue du dextrométhorphan est le O-(2,2,2-trifluoroéthyl)-N-méthylmorphinan.

12. Procédé selon la revendication 1, comprenant en outre l'étape de détermination de ce que le patient va ou non souffrir d'une réaction indésirable vis-à-vis d'un médicament sur la base de la compétence du au moins un système enzymatique.

13. Procédé selon la revendication 1, comprenant en outre l'étape d'établissement d'une première empreinte pour l'activité de compétence du au moins un système enzymatique, de détermination de la concentration du marqueur cible dans l'échantillon de fluide corporel en fonction du temps pour établir une deuxième empreinte, et de détermination de ce que le patient a ou non une maladie sur la base de la comparaison de la première empreinte avec la deuxième empreinte.

14. Procédé selon la revendication 1, comprenant en outre l'étape de détermination de ce que le patient présentera ou non une pathophysiologie sur la base de la compétence du au moins un système enzymatique.

15. Procédé selon la revendication 1, dans lequel l'entité moléculaire parent est apte à être administrée au moyen d'une voie choisie dans le groupe consistant en: sublinguale; gastro-intestinale; vaginale; intraveineuse; sous-cutanée; transdermique; intramusculaire; topique; nasale; et pulmonaire.

16. Procédé selon la revendication 1, comprenant en outre l'étape de construction d'un plan de soins total individualisé pour le patient sur la base de la compétence du au moins un système enzymatique.

17. Procédé selon la revendication 1, dans lequel ledit fluide corporel a été obtenu à partir du dit patient après administration de plus d'une entité moléculaire parent au dit patient; et comprenant en outre l'étape de prescription de dosages bénéfiques pour plus d'un médicament sur la base de la compétence du système enzymatique.

18. Système pour la détermination de la compétence d'un système enzymatique en temps réel au point de traitement, ledit système comprenant:
a) au moins une entité moléculaire parent, tandis qu'au moins un marqueur cible est apte à être généré à partir d'au moins une entité moléculaire parent, et tandis que le marqueur cible est un composé fluoré;
b) un détecteur pour la détection du dit marqueur cible; et
c) un processeur configuré pour enregistrer et analyser des informations fournies par le détecteur;
tandis que
(1) le système enzymatique est le système du cytochrome P450 3A4, l'entité moléculaire parent est un analogue du vérapamil, et le marqueur cible est un métabolite du vérapamil;
ou
(2) le système enzymatique est le système du cytochrome P450 2D6, l'entité moléculaire parent est un analogue du dextrométhorphan, et le marqueur cible est un métabolite du dextrométhorphan.

19. Système selon la revendication 18, dans lequel le processeur est également configuré pour enregistrer une empreinte représentative d'au moins une fonction enzymatique normale; pour déterminer une empreinte d'échantillon sur la base des données fournies par le détecteur; et pour comparer l'empreinte représentative avec l'empreinte d'échantillon.

20. Système selon la revendication 18, dans lequel l'entité moléculaire parent est un substrat pour le au moins un système enzymatique, tandis que l'entité moléculaire parent est choisie dans le groupe consistant en un médicament approuvé par la FDA; un composé de type GRAS; et une nouvelle entité chimique.

21. Système selon la revendication 18, dans lequel le détecteur est choisi dans le groupe consistant en les détecteurs à ensemble métal-isolent-métal (MIME), les arrangements de microdétecteurs optiques à réactivité croisée, les pellicules de polymère fluorescent, la spectroscopie Raman renforcée en surface (SERS), les lasers à diode, les tubes à écoulement ionique sélectionné, les détecteurs à base d'oxyde métallique (MOS), les détecteurs à onde acoustique de volume (BAW), les tubes colorimétriques, la spectroscopie infrarouge, la chromatographie gazeuse, la technologie par détecteur à gaz semi-conducteur; les spectromètres de masse, les spectrophotomètres fluorescents, la technologie par détecteur de gaz à polymère conducteur; la technologie par détecteur aptamère; la technologie par détecteur polymère fluorescent amplificateur (AFP); la technologie par microcantilever; la technologie par pellicule polymère à empreinte moléculaire; les arrangements pour résonance de surface; les détecteurs microgravimétriques; les détecteurs à mode de cisaillement d'épaisseur; ou la technologie par détecteur de gaz à longueur d'onde acoustique de surface.

22. Système selon la revendication 18, dans lequel l'analogue du vérapamil est le N-(2,2,2-trifluoroéthyl)norvérapamil.

23. Système selon la revendication 18, dans lequel l'analogue du dextrométhorphan est le O-(2,2,2-trifluoroéthyl)-N-méthylmorphinan.

24. Kit pour l'évaluation au point de soins de la compétence d'un système enzymatique d'un patient vis-à-vis d'au moins une entité moléculaire parent, ledit kit comprenant:
a) un échantillon de ladite entité moléculaire parent, à partir duquel au moins un marqueur cible est apte à être généré, tandis que le marqueur cible est un composé fluoré; et
b) au moins un détecteur pour la détection du dit marqueur cible;
tandis que
(1) le système enzymatique est le système du cytochrome P450 3A4, l'entité moléculaire parent est un analogue du vérapamil, et le marqueur cible est un métabolite du vérapamil;
ou
(2) le système enzymatique est le système du cytochrome P450 2D6, l'entité moléculaire parent est un analogue du dextrométhorphan, et le marqueur cible est un métabolite du dextrométhorphan.

25. Kit selon la revendication 24, dans lequel le détecteur comprend un moyen de communication qui permet la communication entre le détecteur et un processeur.

26. Kit selon la revendication 25, comprenant en outre un logiciel qui est appliqué au processeur pour utilisation dans l'analyse d'informations procurées par le détecteur.

27. Kit selon la revendication 24, dans lequel le détecteur est choisi dans le groupe consistant en les détecteurs à ensemble métal-isolent-métal (MIME), les arrangements de microdétecteurs optiques à réactivité croisée, les pellicules de polymère fluorescent, la spectroscopie Raman renforcée en surface (SERS), les lasers à diode, les tubes à écoulement ionique sélectionné, les détecteurs à base d'oxyde métallique (MOS), les détecteurs à onde acoustique de volume (BAW), les tubes colorimétriques, la spectroscopie infrarouge, la chromatographie gazeuse, la technologie par détecteur à gaz semi-conducteur; les spectromètres de masse, les spectrophotomètres fluorescents, la technologie par détecteur de gaz à polymère conducteur; la technologie par détecteur aptamère; la technologie par détecteur polymère fluorescent amplificateur (AFP); la technologie par microcantilever; la technologie par pellicule polymère à empreinte moléculaire; les arrangements pour résonance de surface; les détecteurs microgravimétriques; les détecteurs à mode de cisaillement d'épaisseur; ou la technologie par détecteur de gaz à longueur d'onde acoustique de surface.

28. Kit selon la revendication 24, dans lequel l'entité moléculaire parent est un substrat pour un système enzymatique d'intérêt, tandis que l'entité moléculaire parent est choisie dans le groupe consistant en un médicament approuvé par la FDA; un composé de type GRAS; et une nouvelle entité chimique.

29. Kit selon la revendication 24, dans lequel l'analogue du vérapamil est le N-(2,2,2-trifluoroéthyl)norvérapamil.

30. Kit selon la revendication 24, dans lequel l'analogue du dextrométhorphan est le O-(2,2,2-trifluoroéthyl)-N-méthylmorphinan.

31. Procédé pour l'évaluation du caractère approprié potentiel d'un organe d'un patient pour une transplantation, comprenant:
a) exposition *in vitro* d'un détecteur à un fluide corporel provenant d'un patient vis-à-vis duquel doit être déterminée la compétence pour au moins un système enzymatique dans un organe, tandis que ledit détecteur peut distinguer, détecter et quantifier au moins un marqueur cible dans ledit fluide corporel, tandis que ledit au moins un marqueur cible est un composé fluoré, tandis que ledit fluide corporel a été obtenu à partir du dit patient après administration d'au moins une entité moléculaire parent au dit patient, et tandis que ledit au moins un marqueur cible a été généré à partir de ladite au moins une entité moléculaire parent; et
b) sur la base de la présence du marqueur cible, l'établissement de ce que le patient présente ou non une compétence pour le au moins système enzymatique et de ce que l'organe convient ou non pour la transplantation.

32. Procédé pour l'évaluation *in vitro* d'une fonction enzymatique, comprenant:
a) l'administration dans un récipient fermé d'au moins une entité moléculaire parent à un échantillon comprenant au moins une enzyme, tandis que l'activité pour la au moins une enzyme est à déterminer, tandis qu'au moins un marqueur cible est généré à partir de ladite au moins une entité moléculaire parent, tandis que le marqueur cible est un composé fluoré, et tandis que le récipient fermé comprend un espace de tête;
b) l'exposition d'un détecteur à l'espace de tête, tandis que ledit détecteur peut distinguer, détecter et quantifier le marqueur cible dans l'espace de tête; et
c) sur la base de la présence du marqueur cible, l'établissement de ce que la au moins une enzyme est active ou non.
